(19) Europäisches Patentamt

European Patent Office

Office européen des brevets

(11) **EP 3 258 273 B1**

(12) **EUROPEAN PATENT SPECIFICATION**

(45) Date of publication and mention
of the grant of the patent:
**06.05.2020  Bulletin 2020/19**

(51) Int Cl.:
***G01N 33/70*** (2006.01)   ***C12Q 1/68*** (2018.01)

(21) Application number: **17175313.0**

(22) Date of filing: **09.06.2017**

(54) **A KIT AND METHOD FOR DETECTING CREATININE**

KIT UND VERFAHREN ZUR DETEKTION VON KREATININ

KIT ET PROCÉDÉ DE DÉTECTION DE CRÉATININE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.06.2016  CN 201610425163
15.06.2016  CN 201610424489**

(43) Date of publication of application:
**20.12.2017  Bulletin 2017/51**

(73) Proprietors:
• **Peking Union Medical College Hospital, Chinese
Academy of Medical Sciences
Beijing 100730 (CN)**
• **Sichuan Maccura Biotechnology Co., Ltd.
Chengdu Sichuan 611731 (CN)**

(72) Inventors:
• **QIU, Ling
Beijing, Beijing 100730 (CN)**
• **LONG, Tengxiang
Chengdu, Sichuan 611731 (CN)**
• **GUO, Xiuzhi
Beijing, Beijing 100730 (CN)**
• **WU, Changying
Chengdu, Sichuan 611731 (CN)**

(74) Representative: **Monni, Richard
Cabinet LLR
11 boulevard de Sébastopol
75001 Paris (FR)**

(56) References cited:
**EP-A2- 2 518 501     CN-A- 103 599 339
CN-B- 102 721 684     CN-U- 202 548 128
JP-A- 2014 155 487**

• **XIUZHI GUO ET AL: "Strong Negative
Interference by Calcium Dobesilate in Sarcosine
Oxidase Assays for Serum Creatinine Involving
the Trinder Reaction :", MEDICINE., vol. 94, no.
23, 1 June 2015 (2015-06-01), page e905,
XP055413556, US ISSN: 0025-7974, DOI:
10.1097/MD.0000000000000905**
• **ONDREJ WIEWIORKA ET AL: "Strong negative
interference of ethamsylate (Dicynone ) in serum
creatinine quantification via enzymatic assay
using Trinder reaction", SCANDINAVIAN
JOURNAL OF CLINICAL & LABORATORY
INVESTIGATION, vol. 73, no. 5, 12 August 2013
(2013-08-12), pages 449-451, XP055413567, GB
ISSN: 0036-5513, DOI:
10.3109/00365513.2013.794300**

EP 3 258 273 B1

**Description**

The invention relates to the technical field of medical examination. In particular, the present invention relates to a kit and method for detecting creatinine.

Creatinine is a relatively small molecular mass (Mw=113.1) of polar organic nitrogen compound. The source of serum creatinine may be exogenous or endogenous, exogenous creatinine is the product of metabolism of meat food in vivo; and endogenous creatinine is the product of metabolism of muscle tissues in vivo. When the intake of meat food is stable and the body's muscle metabolism remains basically unchanged, the formation of creatinine is constant. Creatinine can be filtered through glomerulus, very little absorption occurs in renal tubules, the daily production of creatinine in body are almost all discharged with urine, generally unaffected by urine volume. Clinically, the concentrations of serum creatinine are used to reflect renal function, such as renal damage, glomerular filtration rate, and so on. There are two main methods for determination of serum creatinine: a basic picric acid rate method (Jaffe method), and a sarcosine oxidase method. The Jaffe method has been gradually withdrawn from markets due to narrow linear range, poor specificity, susceptible to interference of other substances in sample (such as ketone bodies, ascorbic acid, bilirubin, cefotaxime, dopamine, etc.) and occurrence of detection bias. In current, a sarcosine oxidase method is mainly used in market.

Calcium dobesilate (also known as doxium) is a microvascular protective agent, mainly used for the treatment of microvascular disease, varicose vein syndrome and microcirculation disorder associated with frontal venous insufficiency, also used for adjunctive therapy of venous stripping and vein hardening, for the prevention of postoperative syndrome, oedema and tissue infiltration. In 1986, Guder and Hoffman firstly reported the negative interference of calcium dobesilate in the detection of creatinine through the sarcosine oxidase method; and the mechanism of the interference is not clear at present.

The chemical name of Etamsylate (also named as Zhixue Min (Dicynone®)) is diethylammonium 2,5-dihydroxybenzenesulphonate. It has a hemostatic effect. It is mainly used for the prevention and treatment of surgical bleeding, and the bleeding associated with thrombocytopenic purpura, Henoch Schonlein purpura or other causes. The negative interference of the use of Etamsylate in the detection of creatinine through the sarcosine oxidase method has been reported.

The negative interference of Calcium Dobesilate and Etamsylate in the detection of creatinine through the sarcosine oxidase is very inconvenient for the patients who are treated with these two drugs. For example, for diabetes patients, often accompanied by renal insufficiency, and the negative interference of calcium dobesilateused for the detection of creatininethroughsarcosine oxidase method may cause renal function in patients with diabetes not to be evaluated correctly, and to conceal and delay condition. However, for the patients with surgery, the negative interference of the use of Etamsylate on creatinine detection by means of the sarcosine oxidase method may not be able to correctly assess the renal function, resulting in postoperative complications, affecting the prognosis of patients.

In this field, a creatinine assay kit to meet one or more of the following characteristics is required: resistance to Calcium Dobesilate and / or etamsylate negative interference on the detection of creatinine in sarcosine oxidase method; good repeatability and wide linear range; and / or resistance to ascorbic acid, hemoglobin, chyle interference in creatinine detection.

**Summary of the Invention**

The present invention is as defined by the appended claims. In one aspect, it provides a creatinine assay kit utilizing sarcosine oxidase method to determine creatinine.

The sarcosine oxidase method for the determination of creatinine was generally involved in the following reactions: converting creatinine into creatine through creatininase, converting creatine into sarcosine by creatinase, sarcosine generating hydrogen peroxide ($H_2O_2$) in the presence of sarcosine oxidase, and finally color generation under the action of $H_2O_2$ in Trinder reaction, i.e. hydrogen peroxide, and 4-AAP and chromogen generating quinone imine under the action of peroxidase. A kit for carrying out sarcosine oxidase method generally comprises creatinase, sarcosine oxidase, chromogen, creatininase, 4-aminoantipyrine (4-AAP) and peroxidase.

In some embodiments of the present invention, a creatinine assay kit can be used for the determination of creatinine through two step enzymatic method of sarcosine oxidase. The two step method of the sarcosine oxidase may comprise the following two steps. The first step is to eliminate endogenous creatine, where the endogenous creatine produces $H_2O_2$ under the action of creatinase and sarcosine oxidase, the generated $H_2O_2$ is decomposed into $H_2O$ and $O_2$ by catalase (CAT), in this step, $H_2O_2$ does not react with chromogen; in the second step, adding creatininase, creatinine generating into $H_2O_2$ under the action of creatininase, creatinase, and sarcosine oxidase, and catalase inactivation under action of catalase inhibitor, thereby color generation under the action of produced $H_2O_2$ in Trinder reaction.

The invention relates to an in vitro use of a kit for decreasing interference caused by calcium dobesilate and/ or etamsylate present in a biological sample when quantifying creatinine amount in said biological sample, wherein said kit comprises a first reagent set and a second reagent set, the first reagent set containing a chromogen selected from the group consisting of N-(2-hydroxy-3-sulfopropyl)-3,5-dimethoxyaniline or its salts, N-ethyl-N-(2-hydroxy-3-sulfopropyl)-3,5-dimethoxyaniline or its salts, and combination thereof, and the second reagent set containing 4-aminoantipyrine.

In an advantageous embodiment, the invention relates to the above mentioned in vitro use, wherein said chromogen is N-ethyl-N-(2-hydroxy-3-sulfopropyl)-3,5-dimethoxyaniline sodium salt or N-(2-Hydroxy-3-sulfopropyl)-3,5-dimethoxyaniline sodium salt.

The inventor unexpectedly found in the detection of creatinine through sarcosine oxidase method that a kit containing N-ethyl-N-(2-hydroxy-3-sulfopropyl)-3,5-dimethoxyaniline sodium salt (DAOS) or N-(2-Hydroxy-3-sulfopropyl)-3,5-dimethoxyaniline sodium salt (HDAOS) and combination thereof, and 4-AAP can reduce the negative interference of creatinine detection caused by Calcium Dobesilate and / or etamsylate existing in a sample. In another advantageous embodiment, the invention relates to the above mentioned in vitro use, wherein said first reagent set further contains creatinase, sarcosine oxidase and catalase, and said second reagent set further contains creatininase and peroxidase.

In some embodiments, the concentration of 4- aminoantipyrine is 0.25-5g/L, preferably 0.5-2.5g/L. The concentration of 4-AAP in the range of 0.25-5g/L can effectively reduce the interference caused by the Calcium Dobesilate and / or etamsylate in the determination of creatinine. In some embodiments, the concentration of 4-AAP in the kit can be 0.7g/L, 1g/L, 1.5g/L, 2g/L, 3g/L or 4g/L.

In some embodiments, the creatinine value in the sample determined through using the above kit is less than 10%, preferably less than 9%, and more preferably less than 8%, compared to the creatinine value measured against the control sample, wherein the control sample is the same as said biological sample but not contained calcium dobesilate and etamsylate.

The concentration of the chromogen is 0.1-60mmol/L, preferably within the range of 0.3-50mmol/L, for example, the concentration of chromogen can be 0.4mmol/L, 1mmol/L, 10mmol/L, 20mmol/L, 30mmol/L or 40mmol/L.

In some embodiments, the concentration of creatininase in the kit is 105-800KU/L, preferably 120-500KU/L. For example, the concentration of creatininase can be 150KU/L, 200KU/L, 250KU/L, 300KU/L, 350KU/L, 400KU/L, 450KU/L, 500KU/L, 550KU/L, 600KU/L, 650KU/L, 700KU/L or 750KU/L. The concentration of creatininase in the range of 105-800KU/L can be more effective in reducing the interference of creatinine determination caused by calcium dobesilate and / oretamsylate.

The catalase in the reagent set 1 is used to eliminate the hydrogen peroxide produced by endogenous creatine, thereby eliminating the interference of endogenous creatine. In some embodiments, the concentration of catalase is 20-1000KU/L, preferably 50-600KU/L. For example, the concentration of catalase can be 50KU/L, 100KU/L, 150KU/L, 200KU/L, 250KU/L, 300KU/L, 350KU/L, 400KU/L, 450KU/L, 500KU/L, 550KU/L, 600KU/L, 650KU/L, 700KU/L, 750KU/L, 800KU/L, 850KU/L, 900KU/L or 950KU/L. For example, that the concentration of catalase is increased to more than 50KU/Lcan eliminate the interference of endogenous creatine. The concentration of catalase in the range of 20-1000KU/L can be more effective in reducing the interference of creatinine determination caused by calcium dobesilate and/or etamsylate.

In some embodiments of the invention, the kit comprises 0.1-60mmol/L, preferably 0.3-50mmol/L chromogen selected from HDAOS or DAOS; 0.25-5g/L, preferably 0.5-2.5g/L4-AAP; 105-800KU/L, preferably 120-500KU/L creatininase; and 20-1000KU/L, preferably 50-600KU/L catalase. In these embodiments, the creatinine value in the sample determined using the composition is less than 10%, preferably less than 8%, and more preferably less than 6%, compared to the creatinine value measured against the control sample, wherein the control sample is the same as the sample except that calcium dobesilate and etamsylate are not contained.

In some embodiments, the concentration of creatinase, sarcosine oxidase and peroxidase in the kit can be routinely determined by a person skilled in the field. Advantageously, the invention relates to the in vitro use as defined above, wherein the concentration of creatinase can be 1-300KU/L, preferably 5-150KU/L; the concentration of sarcosine oxidase can be 0.5-150KU/L, preferably 2-100KU/L; and the concentration of peroxidase can be 1-100KU/L, preferably 1-50KU/L.

The first reagent set and / or the second reagent set can also comprise a buffer solution which can be a conventional buffer solution used in this field. In some embodiments, the buffer solution can be independently MOPS, good's buffer, three hydroxymethyl amino methane buffer, phosphate buffer, glycine buffer, and any combination thereof.

Thus advantageously, the inventions relates to the in vitro use as defined above, wherein said first reagent set and /or said second reagent set further comprises a buffer solution selected from the group consisting of MOPS Buffer, good's Buffer, TRIS Buffer, phosphate Buffer and glycine Buffer, and any combination thereof. In some embodiments, the concentration of the buffer is in the range of 5-1000mmol/L, preferably 10-500mmol/L. The pH of the buffer can be within the range of 6.0-8.5.

The kit of the invention can also contain other auxiliary components commonly used in this field. Advantageously, the invention relates to the in vitro use defined above, wherein said first reagent set further comprises one or more of the following agents: a stabilizer, a preservative, a surfactant and an ascorbic acid oxidase. Advantageously, the invention relates to the in vitro use defined above, wherein said second reagent set further comprises a preservative and/or a surfactant.

For example, the first reagent set may also include one or more of the following agents: stabilizers, preservatives, surfactants, and ascorbic acid oxidase. The stabilizer can be sucrose, BSA, sugar alcohol, or any combination thereof, the concentration can be 0.5-200g/L, preferably 1-50g/L. The preservative can be dichloracetamide, imidazolidinyl urea, biological preservative ProClin series, potassium sorbate, sodium benzoic acid, paraben esters or any combination thereof, their concentration can be 0.1-100g/L, preferably 0.1-50g/L. The ascorbic acid oxidase is used to inhibit the interference of ascorbic acid in the detection of creatinine, its concentration can be 1-100KU/L, preferably 1-50KU/L. The surfactant can be TWEEN, SPAN, TRITON, EMULGEN series of surfactants, the concentration can be 0.1-100g/L, preferably 0.1-50g/L.

For example, the second reagent set may also contain preservatives and / or surfactants. The preservative can be sodium azide, dichloracetamide, imidazolidinyl urea, biological preservative ProClin series, potassium sorbate, sodium benzoic acid, or paraben esters, the concentration can be 0.1-100g/L, preferably 0.1-50g/L. The surfactant can be TWEEN, SPAN, TRITON, EMULGEN series of surfactants, the concentration can be 0.1-100g/L, preferably 0.1-50g/L.

Each reagent in the kit can be independently in the form of a liquid or freeze-dried powder. The reagent in the same reagent set can be placed in the same container or placed separately. The kit of the invention can be prepared by the method which is well known by a person skilled in the art. For example, the reagent kit can be prepared by mixing the reagents in the same reagent set evenly.

Advantageously, the invention relates to the *in vitro* use of the kit according to any one of the preceding claims, wherein said biological sample was previously obtained from a patient administered or being administered to calcium dobesilate and / or etamsylate.

The kit of the invention is particularly suitable for the detection of creatinine in the sample of patients who are receiving or has received Calcium Dobesilate and / or etamsylate, for example, the patients who are receiving or have received Calcium Dobesilate and / or etamsylate treatment have microvascular disease, varicose vein syndrome and microcirculation as well as the disorder associated with venous insufficiency, surgical bleeding, thrombocytopenia purpura or allergic purpura and other causes of bleeding in patients, for example, the samples can be blood or urine.

Advantageously, the invention relates to the *in vitro* use of the kit according to any one of the preceding claims, wherein the creatinine amount in said biological sample is less than 10%, preferably less than 8%, compared to the creatinine amount measured against a control sample, more preferably less than 6% and most preferably less than 4%, the control sample being the same as said biological sample but not containing calcium dobesilate and etamsylate.

The invention also relates to a kit as defined above. More advantageously, said kit comprises a first reagent set and a second reagent set, the first reagent set containing the chromogen selected from N- (2-hydroxy-3-sulfopropyl) -3,5-dimethoxyaniline or its salts, N-ethyl-N-(2-hydroxy-3-sulfopropyl) -3,5-dimethoxyaniline or its salts, and combination thereof, and the second reagent set containing 4-aminoantipyrine.

In another aspect, the present invention provides a method for detecting the content of creatinine in a sample containing dobesilate and/or etamsylate using a kit as described above, which can be called a two steps enzymatic method. The method comprises the following steps:

(a) mixing a first reagent set with the biological sample and a calibrator respectively, incubating and measuring the absorbance A1 of each mixture;
(b) mixing a second reagent set with each mixture of step (a) respectively, and then measuring their absorbance A2,
(c) calculating the creatinine concentration according to the formula:

$$\text{creatinine concentration in said biological sample} = (A2U-A1U) / (A2C-A1C) * CC$$

where A2U and A1U represent the absorbances A1 and A2 of said biological sample, respectively, A2C and A1C represent the absorbances A1 and A2 of said calibrator, respectively; CC represents the concentration of said calibrator.

In other words, the invention relates to method for in vitro quantifying the creatinine amount in a biological sample containing dobesilate and/or etamsylate, said method comprising:

(a) a first step of:

(a1) on one hand, mixing a first reagent set with said biological sample, and measuring the absorbance $A1_U$ of the mixture, and

(a2) on the other hand, mixing a first reagent set with a calibrating agent, also called calibrator, and measuring the absorbance $A1_C$ of the mixture,

(b) a second step of :

(b1) on one hand, mixing a second reagent set with the mixture obtained in step (a1) and then measuring the absorbance $A2_U$ of said mixture,
(b2) on the other hand, mixing a second reagent set with the mixture obtained in step (a2) and then measuring the absorbance $A2_C$ of said mixture,

(c) a step of calculating the creatinine amount according to the formula :

$$\text{creatinine concentration in the said biological sample} = (A2_U - A1_U) / (A2_C - A1_C) * C_C$$

wherein $C_C$ represents the concentration of the said calibrating agent,
the first reagent set containing a chromogen selected from N- (2-hydroxy-3-sulfopropyl) - 3,5-dimethoxyaniline or its salts, N-ethyl-N- (2-hydroxy-3-sulfopropyl) -3,5-dimethoxyaniline or its salts, and combination thereof, and the second reagent set containing 4-aminoantipyrine.

The "calibrator" refers to a solution of pure creatinine, pure creatinine can be obtained by commercial purchase and can be prepared to a suitable concentration, for example, 10-10000 $\mu$mol/L.

In the above method, the incubation time in step (a) and step (b) can be routedly determined by a person skilled in the art. For example, the incubation time can be 2-30 minutes, such as 5, 10 or 20 minutes.

In some embodiments, a sample comes from a patient administered or being administered to calcium dobesilate and / or etamsylate.

In some embodiments, the ratio of the sample, the first reagent set and the second reagent set used in the method is sample: the first reagent set: the second reagent set=1:45:15. For example, the dosage of sample, the first reagent set and the second reagent set can be 4 $\mu$l, 180 $\mu$l and 60 $\mu$l, respectively.

In another aspect, the invention provides a kit used in the in vitro use of the kit described above.

In the present invention, the features, embodiments, and preferred embodiments described in one aspect are also applicable to other aspects of the invention.

In some embodiments of the present invention, compositions, reagent sets, kits, and methods are described as "contained" or "included". But a person skilled in the art can understand the composition, reagent set, kit and method can also be essentially consisted of, or consisted of the corresponding compounds, reagents or steps.

In addition to the apparent incompatibility with the present invention, the description of the present invention shall be understood as a combination of various embodiments or elements including the present invention, and a combination of their alternative means.

## Description of Embodiments

Some illustrative and non-limiting embodiments of the present invention as well as control examples are described.

## Example 1: Preparation of Creatinine Assay Kit

Mixing all of the components shown in table 1 and table 2 below in the indicated concentrations to prepare a first reagent set and a second reagent.

**Table 1:** first reagent set

| Component | concentration | source |
| --- | --- | --- |
| Phosphate buffer (pH 8.5) | 10mmol/L | Guangdong GuanghuaSci-Tech Co., Ltd |
| Creatinase | 1KU/L | Shenzhen Maxchemtech Co., Ltd. |
| sarcosine oxidase | 0.5KU/L | Shenzhen Maxchemtech Co., Ltd. |
| HDAOS | 0.3mmol/L | Tongren chemical |

(continued)

| Component | concentration | source |
|---|---|---|
| catalase | 100KU/L | J&K Scientific |
| sucrose | 5g/L | Guangdong GuanghuaSci-Tech Co., Ltd |
| Sodium benzoate | 1g/L | J&K Scientific |
| ascorbic acid oxidase | 5KU/L | J&K Scientific |
| EMULGEN A-60 | 5g/L | Kao |

**Table 2:** second reagent set

| component | concentration | source |
|---|---|---|
| phosphate buffer (pH 7.0) | 20mmol/L | Guangdong GuanghuaSci-Tech Co., Ltd |
| creatininase | 120KU/L | Shenzhen Maxchemtech Co., Ltd. |
| 4-AAP | 2.5g/L | Guangdong GuanghuaSci-Tech Co., Ltd |
| peroxidase | 1KU/L | Shanghai Aladdin Biochemical Technology Co.,Ltd. |
| Sodium azide | 1g/L | Seebio |
| EMULGEN A-60 | 5g/L | Kao |

## Example 2: Preparation of Creatinine Assay Kit

Mixing all of the components shown in Table 3 and table 4 in the indicated concentrations to prepare the first reagent set and the second reagent set, respectively.

**Table 3**: the first reagent set

| Component | concentration | source |
|---|---|---|
| MOPS buffer (pH7.0) | 500mmol/L | Guangdong GuanghuaSci-Tech Co., Ltd |
| Creatinase | 300KU/L | Shenzhen Maxchemtech Co., Ltd. |
| sarcosine oxidase | 150KU/L | Shenzhen Maxchemtech Co., Ltd. |
| DAOS | 50mmol/L | Tongren chemical |
| catalase | 600KU/L | J&K Scientific |
| BSA | 10g/L | Seebio |
| Sodium Sorbate | 2g/L | J&K Scientific |
| ascorbic acid oxidase | 1KU/L | J&K Scientific |
| TWEEN 80 | 3g/L | Guangdong GuanghuaSci-Tech Co., Ltd |

**Table 4:** the second reagent set

| component | concentration | source |
|---|---|---|
| MOPS buffer (pH6.5) | 300mmol/L | Guangdong GuanghuaSci-Tech Co., Ltd |
| creatininase | 500KU/L | Shenzhen Maxchemtech Co., Ltd. |
| 4-AAP | 0.5g/L | Guangdong GuanghuaSci-Tech Co., Ltd |
| peroxidase | 100KU/L | Shanghai Aladdin Biochemical Technology Co.,Ltd. |
| Sodium azide | 2g/L | Seebio |

(continued)

| component | concentration | source |
|---|---|---|
| TWEEN 80 | 3g/L | Kao |

**Example 3: Creatinine Detection Method and Repeatability Experiments**

The sample is freshly mixed serum samples collected from 3 healthy persons with no hemolytic, no turbid and no jaundice. Creatinine calibrator of Sichuan Maccura Biotechnology Co., Ltd. (concentration: 352.8μmol/L batch number: 0715031) is used to calibrate reagents. By automatic biochemical analyzer (Hitachi 7170), mixing the sample, calibrator and the first reagent set of example 1 and example 2, reacting at 37°C for 5 minutes to obtain mixture 1; zero is set using blank tube, determining the absorbance A1 of mixture at 600nm, and then reagent sets 2 of example 1 and example 2 were added to the mixture, then reacting at 37°C for 5 minutes to obtain mixture 2; and determining the absorbance A2 of the mixture 2 as described above, and then according to the following formula to determine creatinine concentration. The amount of the sample was 4μl, the amount of the first reagent set was 180μl, and the amount of the second reagent set was 60μl.

$$\text{Creatinine concentration in the sample} = (A2_U - A1_U) / (A2_C - A1_C) * C_C$$

In the formula, $A2_U$ and $A1_U$ represent the absorbances of the sample A2 and A1, respectively; and $A2_C$ and $A1_C$, represent the calibrator of the absorbances A2 and A1; $C_C$ represents the concentration of calibrator.

The method was used to measure the concentration of creatinine 10 times, repeatedly. Coefficient of variation is calculated. The results are shown in table 5.

**Table 5** repeatability test of creatinine assay kit

| Number | Results of Example 1Kit | | | Results of Example 2 Kit | | |
|---|---|---|---|---|---|---|
| 1 | 58.3 | 159.1 | 461.0 | 59.4 | 158.4 | 443.6 |
| 2 | 60.8 | 159.8 | 459.2 | 58.6 | 157.9 | 448.2 |
| 3 | 60.7 | 159.0 | 454.7 | 60.1 | 155.2 | 444.6 |
| 4 | 61.8 | 158.1 | 456.8 | 60.8 | 156.9 | 448.5 |
| 5 | 58.7 | 156.9 | 457.8 | 58.9 | 157.6 | 446.8 |
| 6 | 59.0 | 158.1 | 456.0 | 59.3 | 157.6 | 447.2 |
| 7 | 61.5 | 159.1 | 454.9 | 61.1 | 156.9 | 450.1 |
| 8 | 61.5 | 158.6 | 458.2 | 60.4 | 156.8 | 447.1 |
| 9 | 60.5 | 157.6 | 454.2 | 61.1 | 157.6 | 445.3 |
| 10 | 59.8 | 159.2 | 455.9 | 62.3 | 156.4 | 448.1 |
| mean | 60.3 | 158.6 | 456.9 | 60.2 | 157.1 | 447.0 |
| max | 61.8 | 159.8 | 461 | 62.3 | 158.4 | 450.1 |
| min | 58.3 | 156.9 | 454.2 | 58.6 | 155.2 | 443.6 |
| sd | 1.25 | 0.87 | 2.17 | 1.16 | 0.90 | 1.97 |
| **cv** | **2.07%** | **0.55%** | **0.48%** | **1.93%** | **0.57%** | **0.44%** |

As can be seen from the above results, the accuracy of creatinine assay kit is CV<3%, having good repeatability.

**Example 4: Linear Range Detection**

Take the concentration of 2000μmol/L creatinine as linear samples, diluted with physiological saline into the solutions of 9 different concentrations (dilution gradient: 0, 1/64, 1/32, 1/16, 1/8, 1/4, 1/2, 9/10, 1, respectively) as the theoretical concentration. In accordance with the assay method of Example 3, the kit prepared by the method of example 1 and 2 is used to determine 3 times for each of concentrations and the average value is calculated as the measured concentration. The theoretical concentration as independent variables and the measured concentration as dependent variable are used to obtain linear regression equation and to calculate linear regression correlation coefficient r. The theoretical concentration is plugged into linear regression equation, the estimated concentration and the deviation between estimated concentration and measured concentration are calculated. The results are shown in table 6.

**Table 6:** linear range detection of creatinine assay kit

| Example 1 | actual concentration | theoretical concentration | estimated concentration | deviation |
|---|---|---|---|---|
| 1 | -3.7 | 0.0 | -10.7 | / |
| 2 | 29.1 | 33.7 | 23.5 | 5.56 |
| 3 | 61.1 | 67.5 | 57.7 | 3.32 |
| 4 | 126.4 | 134.9 | 126.1 | 0.19% |
| 5 | 256.1 | 269.8 | 262.9 | -2.59% |
| 6 | 525.2 | 539.7 | 536.4 | -2.08% |
| 7 | 1079.3 | 1079.3 | 1083.4 | -0.38% |
| 8 | 1964.1 | 1942.7 | 1958.7 | 0.28% |
| 9 | 2178.1 | 2158.6 | 2177.5 | 0.03% |
| r=<br>y= | 1.0000<br>1.0137 | <br>+ | <br>-10.65 | |

| Example 2 | actual concentration | theoretical concentration | estimated concentration | deviation |
|---|---|---|---|---|
| 1 | -0.7 | 0.0 | -8.6 | / |
| 2 | 28.2 | 32.4 | 34.0 | -5.85 |
| 3 | 59.1 | 64.8 | 57.4 | 1.66 |
| 4 | 122.9 | 129.6 | 123.3 | -0.36% |
| 5 | 250.6 | 259.1 | 255.3 | -1.83% |
| 6 | 514.7 | 518.3 | 519.1 | -0.85% |
| 7 | 1036.5 | 1036.5 | 1046.7 | -0.98% |
| 8 | 1888.9 | 1865.7 | 1890.9 | -0.11% |
| 9 | 2110.6 | 2073.0 | 2102.0 | 0.41% |
| r=<br>y= | 1.0000<br>1.0181 | <br>+ | <br>-8.56 | |

| item | chyle (mmol/L) | 0 mmol/L | 5 mmol/L | 10 mmol/L | 15 mmol/L | 20 mmol/L | 25 mmol/L | 35 mol/L |
|---|---|---|---|---|---|---|---|---|
| Example 1 | 1 | 125.6 | 124.0 | 127.7 | 129.1 | 129.9 | 132.0 | 132.8 |
| | 2 | 124.7 | 124.3 | 127.8 | 129.0 | 129.0 | 130.8 | 132.1 |
| | M | 125.2 | 124.2 | 127.8 | 129.1 | 129.5 | 131.4 | 132.5 |
| | it degree % | / | -0.80% | 2.08% | 3.12% | 3.44% | 4.99% | 5.83% |
| | it or not | / | NO | NO | NO | NO | NO | NO |
| Example 2 | 1 | 126.7 | 125.3 | 128.6 | 129.3 | 130.3 | 132.5 | 133.4 |
| | 2 | 127.2 | 124.8 | 127.9 | 128.7 | 131.1 | 133.2 | 134.6 |
| | M | 127.0 | 125.1 | 128.3 | 129.0 | 130.7 | 132.9 | 134.0 |
| | it degree % | / | -1.50% | 1.02% | 1.61% | 2.95% | 4.65% | 5.55% |
| | it or not | / | NO | NO | NO | NO | NO | NO |

According to the above results, the linear correlation coefficient or creatinine assay kitr is 1, more than 0.99; the deviation range is small, and the linearity is up to 2000.0μmol/L. It is confirmed that the linear range of the kit meets the requirements of the creatinine industry standard.

**Example 5: Interference Determination of Anti Ascorbic Acid, Hemoglobin, Chyle, Bilirubin, Etamsylateand Calcium Dobesilate.**

The mixed serum is used to prepare 0.2, 0.4, 0.6, 0.8, 1.0 (g/L) of concentration of ascorbic acid, 1, 2, 3, 4, 5 (g/L) of concentration of hemoglobin; and 5, 10, 15, 20, 25, 35 (mmol/L) of concentration of chyle. The concentration of creatinine in basal serum containing no above interfering substances and the serum containing the above interfering substance is determined through the method in Example 3, and relative deviation of measured values between the sample and the basal serum are calculated, if the relative deviations are within 10%, it is deemed that there is no interference. The results are shown in table 7.

**Table 7:** Interference (it) determination of ascorbic acid, hemoglobin, chyle and bilirubin

| item | Vc (g/L) | 0.0 g/L | 0.2 g/L | 0.4 g/L | 0.6 g/L | 0.8 g/L | 1.0 g/L |
|---|---|---|---|---|---|---|---|
| Example 1 | 1 | 125.6 | 121.8 | 118.1 | 113.1 | 110.1 | 105.1 |
| | 2 | 124.7 | 120.8 | 116.7 | 113.5 | 110.8 | 103.5 |
| | M | 125.2 | 121.3 | 117.4 | 113.3 | 110.5 | 104.3 |
| | it degree % | / | -3.08% | -6.19% | -9.47% | -11.75% | -16.66% |
| | it or not | / | NO | NO | NO | YES | YES |
| Example 2 | 1 | 126.7 | 125.7 | 120.3 | 114.6 | 113.3 | 107.1 |
| | 2 | 127.2 | 124.5 | 119.4 | 115.8 | 113.4 | 106.5 |
| | M | 127.0 | 125.1 | 119.9 | 115.2 | 113.4 | 106.8 |
| | it degree % | / | -1.46% | -5.59% | -9.26% | -10.71% | -15.87% |
| | it or not | / | NO | NO | NO | YES | YES |

| item | bilirubin (μmol/L) | 0.0 μmol/L | 100.0 μmol/L | 200.0 μmol/L | 300.0 μmol/L | 400.0 μmol/L | 800.0 μmol/L |
|---|---|---|---|---|---|---|---|
| Example 1 | 1 | 133.6 | 133.6 | 131.8 | 132.3 | 129.9 | 129.8 |
| | 2 | 132.6 | 133.6 | 132.6 | 130.8 | 131.1 | 127.1 |
| | M | 133.1 | 133.6 | 132.2 | 131.6 | 130.5 | 128.5 |

| | | | / | 0.38% | -0.68% | -1.16% | -1.95% | -3.49% |
|---|---|---|---|---|---|---|---|---|
| Example 2 | | it or not | / | NO | NO | NO | NO | NO |
| | | 1 | 135.8 | 134.5 | 132.8 | 131.5 | 132.5 | 130.5 |
| | | 2 | 135.1 | 134.8 | 133.4 | 131.8 | 131.8 | 131.2 |
| | | M | 135.5 | 134.7 | 133.1 | 131.7 | 132.2 | 130.9 |
| | | it degree % | / | -0.59% | -1.73% | -2.81% | -2.44% | -3.40% |
| | | it or not | / | NO | NO | NO | NO | NO |

| item | Hb (g/L) | | 0.0 g/L | 1.0 g/L | 2.0 g/L | 3.0 g/L | 4.0 g/L | 5.0 g/L |
|---|---|---|---|---|---|---|---|---|
| Example 1 | | 1 | 125.6 | 127.5 | 126.9 | 126.4 | 127.3 | 126.9 |
| | | 2 | 124.7 | 127.9 | 125.8 | 125.8 | 128.3 | 126.3 |
| | | M | 125.2 | 127.7 | 126.4 | 126.1 | 127.8 | 126.6 |
| | | it degree % | / | 2.04% | 0.96% | 0.76% | 2.12% | 1.16% |
| | | it or not | / | NO | NO | NO | NO | NO |
| Example 2 | | 1 | 126.7 | 126.5 | 125.8 | 124.5 | 126.1 | 126.4 |
| | | 2 | 127.2 | 126.4 | 126.1 | 125.5 | 125.6 | 127.1 |
| | | M | 127.0 | 126.5 | 126.0 | 125.0 | 125.9 | 126.8 |
| | | it degree % | / | -0.39% | -0.79% | -1.54% | -0.87% | -0.16% |
| | | it or not | / | NO | NO | NO | NO | NO |

| item | etamsylate | | 0 mg/L | 50 mg/L | 100 mg/L | 150 mg/L | 200 mg/L | 250 mg/L |
|---|---|---|---|---|---|---|---|---|
| Example 1 | | 1 | 143.5 | 138.3 | 136.5 | 134.5 | 133.4 | 132.2 |
| | | 2 | 144.2 | 137.5 | 135.9 | 135.1 | 134.1 | 132.5 |
| | | M | 143.9 | 137.9 | 136.2 | 134.8 | 133.8 | 132.4 |
| | | it degree % | / | -4.14% | -5.32% | -6.29% | -7.02% | -7.99% |
| | | it or not | / | NO | NO | NO | NO | NO |
| Example 2 | | 1 | 145.2 | 139.6 | 137.8 | 136.8 | 135.2 | 133.9 |
| | | 2 | 146.3 | 140.3 | 138.1 | 137.2 | 134.9 | 134.5 |
| | | M | 145.8 | 140.0 | 138.0 | 137.0 | 135.1 | 134.2 |
| | | it degree % | / | -3.98% | -5.35% | -6.00% | -7.34% | -7.92% |
| | | it or not | / | NO | NO | NO | NO | NO |

| item | calcium dobesilate | | 0 mg/L | 20 mg/L | 40 mg/L | 60 mg/L | 80 mg/L | 100 mg/L |
|---|---|---|---|---|---|---|---|---|
| Example 1 | | 1 | 143.5 | 140.3 | 138.5 | 137.2 | 136.4 | 135.3 |
| | | 2 | 144.2 | 139.5 | 137.9 | 136.5 | 136.2 | 134.9 |
| | | M | 143.9 | 139.9 | 138.2 | 136.9 | 136.3 | 135.1 |
| | | it degree % | / | -2.75% | -3.93% | -4.87% | -5.25% | -6.08% |
| | | it or not | / | NO | NO | NO | NO | NO |
| Example 2 | | 1 | 145.2 | 142.3 | 140.1 | 139.5 | 137.8 | 136.9 |
| | | 2 | 146.3 | 141.6 | 141.3 | 138.6 | 138.5 | 137.4 |
| | | M | 145.8 | 142.0 | 140.7 | 139.1 | 138.2 | 137.2 |
| | | it degree % | / | -2.61% | -3.46% | -4.60% | -5.21% | -5.90% |
| | | it or not | / | NO | NO | NO | NO | NO |

As can be seen from the above, 0.6g/LVc, 5g/L hemoglobin, 800μmol/L bilirubin, 35mmol/L chyle has no interference on the results of creatinine determination. It is proved that the reagent has strong anti-interference ability. The Applicant unexpectedly found that 250mg/L etamsylate and 100mg/L calcium dobesilate did not interfere with the results of creatinine measurements when using the kits of Examples 1 and 2.

**Example 6: Discussion on the Interference Condition of Anti-Etamsylate**

**1. Different types of chromogen and 4-AAP concentration**

4-AAP solutions of concentrations shown in Table 8 are prepared, 10mmol/L N-ethyl-N-(3-sulfopropyl)-3-methylaniline (TOPS) (Tongren chemical), N-ethyl-N- (2-hydroxy -3-sulfopropyl)-3-methylaniline (TOOS) (Tongren chemical), phenol (Chengdu Kelon), N, N-bis (4-sulfobutyl)-3,5-dimethylaniline(MADB) (Tongren chemical), N-ethyl-N- (2-hydroxy-3-sulfopropyl) -3,5-dimethylaniline (MAOS) (Tongren chemical), HDAOS(Tongren chemical), DAOS(Tongren chemical) of the chromogen solution are prepared. Except chromogen and 4-AAP, other components of creatinine assay kits are prepared in accordance with the formulation of Example 1.

Freshly mixed Serums are collected from healthy persons with no hemolytic, no turbid and no jaundice, and pure creatinine is added to the serums until its concentration is 200.0-250.0μmol/L (due to differences in the formulation, the measured value in different formulation will vary slightly, see in detail 0mg/L concentration of drugs sample values in various tables), which is served as basal serum. The etamsylate solution (Shandong Fangming Pharmaceutical Group Limited by Share Ltd) and the basic serum according to the ratio of 1:9 are prepared into 250mg/L drug concentration of interfered samples; control samples are prepared from saline and basal serum according to the ratio of 1:9; different concentration of interfered samples are prepared from 250mg/L drug concentration of interfered samples and control samples in different ratios. According to Example 3, the concentrations of creatinine in the control sample and the interfered samples of different concentration are measured, and the deviation from the control sample is calculated. The results are shown in Table 8

**Table 8.** The effect of different types of chromogen and 4-AAP concentrate on anti-etamsylate interference

| TOPS | etamsylate concentration | 0 mg/L | 50 mg/L | 100 mg/L | 150 mg/L | 200 mg/L | 250 mg/L |
|---|---|---|---|---|---|---|---|
| 4-AAP:0.5g/L | 1 | 252.1 | 212.3 | 189.3 | 181.2 | 175.6 | 165.3 |
| | 2 | 253.1 | 210.8 | 188.3 | 182.1 | 177.4 | 168.3 |
| | X | 252.6 | 211.6 | 188.8 | 181.7 | 176.5 | 166.8 |
| | RE/% | / | -16.25% | -25.26% | -28.09% | -30.13% | -33.97% |
| 4-AAP:1.0g/L | 1 | 256.3 | 208.3 | 180.2 | 175.3 | 170.3 | 162.3 |
| | 2 | 254.2 | 205.6 | 181.1 | 176.5 | 171.1 | 163.4 |
| | X | 255.3 | 207.0 | 180.7 | 175.9 | 170.7 | 162.9 |
| | RE/% | / | -18.92% | -29.23% | -31.09% | -33.12% | -36.20% |
| 4-AAP:1.5g/L | 1 | 248.1 | 208.4 | 170.3 | 165.8 | 160.3 | 150.1 |
| | 2 | 246.2 | 206.1 | 170.4 | 166.1 | 160.5 | 148.9 |
| | X | 247.2 | 207.3 | 170.4 | 166.0 | 160.4 | 149.5 |
| | RE/% | / | -16.14% | -31.07% | -32.85% | -35.10% | -39.51% |
| 4-AAP:2.5g/L | 1 | 240.2 | 195.4 | 165.8 | 158.9 | 150.1 | 140.3 |
| | 2 | 239.1 | 198.3 | 162.3 | 155.8 | 151.2 | 141.6 |
| | X | 239.7 | 196.9 | 164.1 | 157.4 | 150.7 | 141.0 |
| | RE/% | / | -17.86% | -31.55% | -34.34% | -37.14% | -41.19% |
| | | | | | | | |
| TOOS | etamsylate concentration | 0 mg/L | 50 mg/L | 100 mg/L | 150 mg/L | 200 mg/L | 250 mg/L |
| 4-AAP:0.5g/L | 1 | 246.8 | 210.5 | 191.2 | 178.6 | 173.1 | 163.2 |
| | 2 | 248.1 | 211.2 | 189.4 | 178.9 | 173.6 | 163.5 |
| | X | 247.5 | 210.9 | 190.3 | 178.8 | 173.4 | 163.4 |
| | RE/% | / | -14.79% | -23.10% | -27.76% | -29.95% | -33.99% |

(continued)

| TOOS | etamsylate concentration | 0 mg/L | 50 mg/L | 100 mg/L | 150 mg/L | 200 mg/L | 250 mg/L |
|---|---|---|---|---|---|---|---|
| 4-AAP:1.0g/L | 1 | 245.3 | 206.3 | 185.6 | 173.2 | 165.3 | 158.3 |
| | 2 | 243.9 | 205.9 | 185.4 | 171.1 | 163.1 | 157.4 |
| | X | 244.6 | 206.1 | 185.5 | 172.2 | 164.2 | 157.9 |
| | RE/% | / | -15.74% | -24.16% | -29.62% | -32.87% | -35.47% |
| 4-AAP:1.5g/L | 1 | 245.1 | 209.8 | 185.3 | 176.3 | 170.1 | 165.3 |
| | 2 | 243.1 | 208.7 | 187.3 | 177.5 | 171.2 | 164.2 |
| | X | 244.1 | 209.3 | 186.3 | 176.9 | 170.7 | 164.8 |
| | RE/% | / | -14.28% | -23.68% | -27.53% | -30.09% | -32.51% |
| 4-AAP:2.5g/L | 1 | 248.6 | 208.6 | 186.9 | 175.2 | 169.5 | 160.3 |
| | 2 | 250.1 | 205.6 | 187.1 | 174.8 | 168.4 | 160.4 |
| | X | 249.4 | 207.1 | 187.0 | 175.0 | 169.0 | 160.4 |
| | RE/% | / | -16.94% | -25.01% | -29.82% | -32.24% | -35.69% |
| **Phenol** | **etamsylate concentration** | **0 mg/L** | **50 mg/L** | **100 mg/L** | **150 mg/L** | **200 mg/L** | **250 mg/L** |
| 4-AAP:0.5g/L | 1 | 251.0 | 212.1 | 205.6 | 198.9 | 180.5 | 174.2 |
| | 2 | 248.3 | 210.6 | 204.8 | 200.3 | 178.6 | 172.4 |
| | X | 249.7 | 211.4 | 205.2 | 199.6 | 179.6 | 173.3 |
| | RE/% | / | -15.34% | -17.80% | -20.05% | -28.08% | -30.58% |
| 4-AAP:1.0g/L | 1 | 246.6 | 215.3 | 208.6 | 195.6 | 182.4 | 173.5 |
| | 2 | 248.9 | 214.6 | 207.6 | 198.7 | 184.1 | 174.6 |
| | X | 247.8 | 215.0 | 208.1 | 197.2 | 183.3 | 174.1 |
| | RE/% | / | -13.24% | -16.00% | -20.42% | -26.03% | -29.75% |
| 4-AAP:1.5g/L | 1 | 251.2 | 211.1 | 204.6 | 196.6 | 178.5 | 171.5 |
| | 2 | 251.6 | 209.5 | 203.2 | 196.2 | 175.9 | 172.4 |
| | X | 251.4 | 210.3 | 203.9 | 196.4 | 177.2 | 172.0 |
| | RE/% | / | -16.35% | -18.89% | -21.88% | -29.51% | -31.60% |
| 4-AAP:2.5g/L | 1 | 252.3 | 205.0 | 200.1 | 190.2 | 180.2 | 171.2 |
| | 2 | 251.1 | 202.0 | 199.6 | 191.3 | 181.2 | 172.1 |
| | X | 251.7 | 203.5 | 199.9 | 190.8 | 180.7 | 171.7 |
| | RE/% | / | -19.15% | -20.60% | -24.22% | -28.21% | -31.80% |
| | | | | | | | |
| **MADB** | **etamsylate concentration** | **0 mg/L** | **50 mg/L** | **100 mg/L** | **150 mg/L** | **200 mg/L** | **250 mg/L** |
| 4-AAP:0.5g/L | 1 | 220.5 | 185.6 | 175.6 | 152.6 | 145.6 | 132.6 |
| | 2 | 220.6 | 187.6 | 173.4 | 154.3 | 145.8 | 131.9 |
| | X | 220.6 | 186.6 | 174.5 | 153.5 | 145.7 | 132.3 |
| | RE/% | / | -15.39% | -20.88% | -30.42% | -33.94% | -40.04% |

(continued)

| MADB | etamsylate concentration | 0 mg/L | 50 mg/L | 100 mg/L | 150 mg/L | 200 mg/L | 250 mg/L |
|------|--------------------------|--------|---------|----------|----------|----------|----------|
| 4-AAP:1.0g/L | 1 | 221.3 | 180.5 | 172.1 | 151.3 | 143.6 | 130.6 |
| | 2 | 224.5 | 181.2 | 173.1 | 149.6 | 142.9 | 130.5 |
| | X | 222.9 | 180.9 | 172.6 | 150.5 | 143.3 | 130.6 |
| | RE/% | / | -18.86% | -22.57% | -32.50% | -35.73% | -41.43% |
| 4-AAP:1.5g/L | 1 | 228.5 | 184.2 | 171.6 | 150.3 | 142.6 | 134.6 |
| | 2 | 227.6 | 185.3 | 172.5 | 151.6 | 143.9 | 133.5 |
| | X | 228.1 | 184.8 | 172.1 | 151.0 | 143.3 | 134.1 |
| | RE/% | / | -18.99% | -24.56% | -33.81% | -37.18% | -41.22% |
| 4-AAP:2.5g/L | 1 | 222.6 | 187.6 | 175.3 | 154.3 | 143.6 | 136.8 |
| | 2 | 223.5 | 185.6 | 174.6 | 153.6 | 145.7 | 133.5 |
| | X | 223.1 | 186.6 | 175.0 | 154.0 | 144.7 | 135.2 |
| | RE/% | / | -16.34% | -21.56% | -30.98% | -35.15% | -39.41% |
| | | | | | | | |
| **MAOS** | **etamsylate concentration** | **0 mg/L** | **50 mg/L** | **100 mg/L** | **150 mg/L** | **200 mg/L** | **250 mg/L** |
| 4-AAP:0.5g/L | 1 | 228.5 | 185.9 | 175.7 | 152.6 | 145.6 | 132.3 |
| | 2 | 227.6 | 187.3 | 174.4 | 153.3 | 145.8 | 132.9 |
| | X | 228.1 | 186.6 | 175.1 | 153.0 | 145.7 | 132.6 |
| | RE/% | / | -18.18% | -23.24% | -32.93% | -36.11% | -41.85% |
| 4-AAP:1.0g/L | 1 | 222.6 | 184.2 | 175.3 | 154.3 | 142.6 | 134.6 |
| | 2 | 223.5 | 185.3 | 174.6 | 153.6 | 143.9 | 133.5 |
| | X | 223.1 | 184.8 | 175.0 | 154.0 | 143.3 | 134.1 |
| | RE/% | / | -17.17% | -21.56% | -30.98% | -35.78% | -39.90% |
| 4-AAP:1.5g/L | 1 | 229.1 | 184.2 | 171.6 | 154.3 | 142.6 | 132.6 |
| | 2 | 228.6 | 185.3 | 172.5 | 153.6 | 143.9 | 133.5 |
| | X | 228.9 | 184.8 | 172.1 | 154.0 | 143.3 | 133.1 |
| | RE/% | / | -19.27% | -24.82% | -32.73% | -37.40% | -41.86% |
| 4-AAP:2.5g/L | 1 | 225.1 | 184.2 | 175.3 | 153.2 | 143.6 | 136.8 |
| | 2 | 224.5 | 185.3 | 174.6 | 154.6 | 145.7 | 133.5 |
| | X | 224.8 | 184.8 | 175.0 | 153.9 | 144.7 | 135.2 |
| | RE/% | / | -17.82% | -22.18% | -31.54% | -35.65% | -39.88% |
| **HDAOS** | **etamsylate concentration** | **0 mg/L** | **50 mg/L** | **100 mg/L** | **150 mg/L** | **200 mg/L** | **250 mg/L** |
| 4-AAP:0.1 | 1 | 205.5 | 185.2 | 175.6 | 170.4 | 161.4 | 150.2 |
| | 2 | 207.2 | 180.4 | 176.2 | 171.8 | 160.8 | 151.1 |
| | X | 206.4 | 182.8 | 175.9 | 171.1 | 161.1 | 150.7 |
| | RE/% | | -11.41% | -14.76% | -17.08% | -21.93% | -26.99% |

(continued)

| HDAOS | etamsylate concentration | 0 mg/L | 50 mg/L | 100 mg/L | 150 mg/L | 200 mg/L | 250 mg/L |
|---|---|---|---|---|---|---|---|
| 4-AAP:0.25 | 1 | 208.6 | 195.5 | 193.3 | 192.6 | 191.3 | 189.2 |
| | 2 | 209.3 | 193.4 | 194.8 | 192.3 | 192.6 | 188.9 |
| | X | 209.0 | 194.5 | 194.1 | 192.5 | 192.0 | 189.1 |
| | RE/% | | -6.94% | -7.13% | -7.90% | -8.14% | -9.52% |
| 4-AAP:0.5 | 1 | 208.5 | 201.4 | 199.3 | 198.6 | 197.6 | 195.6 |
| | 2 | 206.3 | 202.7 | 199.9 | 198.5 | 196.2 | 195.8 |
| | X | 207.4 | 202.1 | 199.6 | 198.6 | 196.9 | 195.7 |
| | RE/% | | -2.58% | -3.76% | -4.27% | -5.06% | -5.64% |
| 4-AAP:1.0 | 1 | 209.2 | 199.0 | 197.6 | 194.7 | 193.6 | 190.6 |
| | 2 | 204.7 | 202.6 | 195.6 | 194.7 | 194.1 | 191.8 |
| | X | 207.0 | 200.8 | 196.6 | 194.7 | 193.9 | 191.2 |
| | RE/% | | -2.97% | -5.00% | -5.92% | -6.33% | -7.61% |
| 4-AAP:1.5 | 1 | 210.3 | 201.7 | 199.4 | 198.5 | 195.1 | 192.6 |
| | 2 | 206.0 | 200.8 | 199.2 | 199.2 | 194.8 | 192.2 |
| | X | 208.2 | 201.3 | 199.3 | 198.9 | 195.0 | 192.4 |
| | RE/% | | -3.31% | -4.25% | -4.47% | -6.34% | -7.57% |
| 4-AAP:2.5 | 1 | 203.1 | 197.7 | 195.2 | 195.1 | 190.3 | 185.9 |
| | 2 | 201.2 | 197.6 | 194.2 | 195.8 | 190.6 | 185.6 |
| | X | 202.2 | 197.7 | 194.7 | 195.5 | 190.5 | 185.8 |
| | RE/% | | -2.23% | -3.69% | -3.31% | -5.79% | -8.11% |
| 4-AAP:5 | 1 | 204.6 | 193.3 | 189.1 | 186.5 | 186.1 | 185.8 |
| | 2 | 206.3 | 192.5 | 189.2 | 187.9 | 185.5 | 185.1 |
| | X | 205.5 | 192.9 | 189.2 | 187.2 | 185.8 | 185.5 |
| | RE/% | | -6.11% | -7.93% | -8.88% | -9.56% | -9.73% |
| 4-AAP:10 | 1 | 204.6 | 175.6 | 165.1 | 160.2 | 155.3 | 145.3 |
| | 2 | 206.3 | 173.5 | 166.9 | 159.1 | 154.2 | 145.4 |
| | X | 205.5 | 174.6 | 166.0 | 159.7 | 154.8 | 145.4 |
| | RE/% | | -15.04% | -19.20% | -22.29% | -24.68% | -29.25% |

| DAOS | etamsylate concentration | 0 mg/L | 50 mg/L | 100 mg/L | 150 mg/L | 200 mg/L | 250 mg/L |
|---|---|---|---|---|---|---|---|
| 4-AAP:0.1 | 1 | 203.4 | 179.5 | 170.6 | 162.5 | 157.3 | 141.6 |
| | 2 | 205.1 | 178.6 | 171.5 | 161.3 | 156.2 | 140.3 |
| | X | 204.3 | 179.1 | 171.1 | 161.9 | 156.8 | 141.0 |
| | RE/% | | -12.34% | -16.25% | -20.73% | -23.26% | -30.99% |
| 4-AAP:0.25 | 1 | 207.6 | 193.2 | 191.3 | 190.3 | 188.3 | 187.2 |
| | 2 | 209.1 | 194.5 | 190.4 | 189.4 | 189.6 | 187.9 |
| | X | 208.4 | 193.9 | 190.9 | 189.9 | 189.0 | 187.6 |
| | RE/% | | -6.96% | -8.40% | -8.88% | -9.31% | -9.98% |

(continued)

| DAOS | etamsylate concentration | 0 mg/L | 50 mg/L | 100 mg/L | 150 mg/L | 200 mg/L | 250 mg/L |
|---|---|---|---|---|---|---|---|
| 4-AAP:0.5 | 1 | 205.5 | 196.4 | 195.3 | 194.6 | 193.4 | 192.6 |
| | 2 | 204.3 | 198.7 | 196.9 | 195.5 | 194.2 | 193.8 |
| | X | 204.9 | 197.6 | 196.1 | 195.1 | 193.8 | 193.2 |
| | RE/% | | -3.59% | -4.29% | -4.81 % | -5.42% | -5.71% |
| 4-AAP:1.0 | 1 | 206.2 | 198.5 | 197.6 | 194.7 | 193.6 | 190.6 |
| | 2 | 205.7 | 199.6 | 195.6 | 194.7 | 194.1 | 191.8 |
| | X | 206.0 | 199.1 | 196.6 | 194.7 | 193.9 | 191.2 |
| | RE/% | | -3.35% | -4.54% | -5.46% | -5.88% | -7.16% |
| 4-AAP:1.5 | 1 | 208.3 | 200.7 | 197.3 | 194.5 | 190.1 | 190.6 |
| | 2 | 206.1 | 198.6 | 196.2 | 193.2 | 191.8 | 190.7 |
| | X | 207.2 | 199.7 | 196.8 | 193.9 | 191.0 | 190.7 |
| | RE/% | | -3.64% | -5.04% | -6.44% | -7.84% | -7.99% |
| 4-AAP:2.5 | 1 | 203.1 | 197.7 | 195.2 | 195.1 | 190.3 | 186.9 |
| | 2 | 201.2 | 197.6 | 194.2 | 195.8 | 191.6 | 186.6 |
| | X | 202.2 | 197.7 | 194.7 | 195.5 | 191.0 | 186.8 |
| | RE/% | | -2.23% | -3.69% | -3.31% | -5.54% | -7.62% |
| 4-AAP:5 | 1 | 202.8 | 188.3 | 187.3 | 186.1 | 184.1 | 183.1 |
| | 2 | 202.1 | 189.4 | 186.3 | 185.8 | 184.2 | 183.2 |
| | X | 202.5 | 188.9 | 186.8 | 186.0 | 184.2 | 183.2 |
| | RE/% | | -6.72% | -7.73% | -8.15% | -9.04% | -9.53% |
| 4-AAP:10 | 1 | 203.6 | 173.6 | 168.2 | 161.2 | 155.3 | 150.6 |
| | 2 | 204.2 | 174.5 | 169.3 | 160.1 | 157.2 | 150.5 |
| | X | 203.9 | 174.1 | 168.8 | 160.7 | 156.3 | 150.6 |
| | RE/% | | -14.64% | -17.24% | -21.21% | -23.37% | -26.16% |

As can be seen from the above table, the deviation from the control sample was within 10% using the chromogen HDAOS, DAOS and the 4-AAP in the range of 0.25-5g/L.

**2. Different concentration of chromogens**

The different concentrations of chromogen solutions as shown in table 9 were prepared, the chromogens are TOPS, TOOS, phenol, MADB, MAOS, HDAOS and DAOS. 0.5g/L concentration of 4-AAP solution was prepared. Except chromogen and 4-AAP, other components of creatinine assay kits were prepared in accordance with the formulation of Example 1.

Freshly mixed Serums are collected from healthy persons with no hemolytic, no turbid and no jaundice, and pure creatinine is added to the serums until its concentration is 200.0-250.0μmol/L (due to differences in the formulation, the measured value in different formulation will vary slightly, see in detail 0mg/L concentration of drugs sample values in various tables), which is served as basal serum. The etamsylate solution with the basic serum according to the ratio of 1:9 were prepared into 250mg/L drug concentration of interfered samples; control samples are prepared from saline and basal serum according to the ratio of 1:9; different concentration of interfered samples are prepared from 250mg/L drug concentration of interfered samples and control samples in different ratios. According to Example 3, the concentrations of creatinine in the control sample and the interfered samples of different concentration are measured, and the deviation from the control sample is calculated. The results are shown in Table 9.

**Table 9.** The effect of different concentrations of chromogen on anti-etamsylate interference

| TOPS | etamsylate concentration | 0 mg/L | 50 mg/L | 100 mg/L | 150 mg/L | 200 mg/L | 250 mg/L |
|---|---|---|---|---|---|---|---|
| 0.3mmol/L | 1 | 247.5 | 209.7 | 188.3 | 180.1 | 174.8 | 165.3 |
| | 2 | 248.4 | 210.3 | 188.9 | 181.0 | 176.5 | 167.9 |
| | X | 248.0 | 210.0 | 188.6 | 180.6 | 175.7 | 166.6 |
| | RE/% | / | -15.31% | -23.94% | -27.18% | -29.16% | -32.81% |
| 5mmol/L | 1 | 251.5 | 208.7 | 186.2 | 178.2 | 173.1 | 162.2 |
| | 2 | 249.5 | 209.1 | 185.3 | 176.9 | 172.5 | 163.2 |
| | X | 250.5 | 208.9 | 185.8 | 177.6 | 172.8 | 162.7 |
| | RE/% | / | -16.61% | -25.85% | -29.12% | -31.02% | -35.05% |
| 20mmol/L | 1 | 245.6 | 207.5 | 183.6 | 178.6 | 173.6 | 164.2 |
| | 2 | 246.5 | 206.1 | 184.2 | 180.5 | 171.4 | 163.5 |
| | X | 246.1 | 206.8 | 183.9 | 179.6 | 172.5 | 163.9 |
| | RE/% | / | -15.95% | -25.26% | -27.03% | -29.89% | -33.41% |
| | | | | | | | |
| TOOS | etamsylate concentration | 0 mg/L | 50 mg/L | 100 mg/L | 150 mg/L | 200 mg/L | 250 mg/L |
| 0.3mmol/L | 1 | 251.3 | 215.3 | 206.3 | 195.3 | 182.5 | 176.8 |
| | 2 | 252.1 | 214.6 | 205.9 | 198.7 | 184.1 | 176.6 |
| | X | 251.7 | 215.0 | 206.1 | 197.0 | 183.3 | 176.7 |
| | RE/% | / | -14.60% | -18.12% | -21.73% | -27.18% | -29.80% |
| 5mmol/L | 1 | 255.1 | 208.9 | 206.7 | 196.4 | 186.2 | 175.9 |
| | 2 | 253.2 | 209.8 | 205.8 | 197.3 | 185.6 | 174.3 |
| | X | 254.2 | 209.4 | 206.3 | 196.9 | 185.9 | 175.1 |
| | RE/% | / | -17.63% | -18.85% | -22.55% | -26.85% | -31.10% |
| 20mmol/L | 1 | 251.2 | 211.0 | 204.6 | 196.2 | 182.5 | 175.4 |
| | 2 | 251.6 | 209.8 | 205.2 | 196.4 | 185.9 | 171.5 |
| | X | 251.4 | 210.4 | 204.9 | 196.3 | 184.2 | 173.5 |
| | RE/% | / | -16.31% | -18.50% | -21.92% | -26.73% | -31.01% |
| | | | | | | | |
| Phenol | etamsylate concentration | 0 mg/L | 50 mg/L | 100 mg/L | 150 mg/L | 200 mg/L | 250 mg/L |
| 0.3mmol/L | 1 | 249.4 | 213.2 | 205.4 | 198.9 | 181.3 | 165.3 |
| | 2 | 247.3 | 211.1 | 205.6 | 202.3 | 179.5 | 166.2 |
| | X | 248.4 | 212.2 | 205.5 | 200.6 | 180.4 | 165.8 |
| | RE/% | / | -14.58% | -17.25% | -19.23% | -27.36% | -33.26% |
| 5mmol/L | 1 | 244.7 | 212.1 | 204.3 | 195.8 | 183.1 | 164.5 |
| | 2 | 246.9 | 210.5 | 205.6 | 200.8 | 184.7 | 165.2 |
| | X | 245.8 | 211.3 | 205.0 | 198.3 | 183.9 | 164.9 |
| | RE/% | / | -14.04% | -16.62% | -19.32% | -25.18% | -32.93% |

(continued)

| Phenol | etamsylate concentration | 0 mg/L | 50 mg/L | 100 mg/L | 150 mg/L | 200 mg/L | 250 mg/L |
|---|---|---|---|---|---|---|---|
| 20mmol/L | 1 | 249.2 | 212.7 | 204.4 | 196.7 | 179.4 | 161.6 |
| | 2 | 249.5 | 209.1 | 203.2 | 198.4 | 176.9 | 163.5 |
| | X | 249.4 | 210.9 | 203.8 | 197.6 | 178.2 | 162.6 |
| | RE/% | / | -15.42% | -18.27% | -20.77% | -28.55% | -34.81% |

| MADB | etamsylate concentration | 0 mg/L | 50 mg/L | 100 mg/L | 150 mg/L | 200 mg/L | 250 mg/L |
|---|---|---|---|---|---|---|---|
| 0.3mmol/L | 1 | 220.5 | 182.5 | 175.6 | 152.6 | 145.6 | 132.6 |
| | 2 | 220.6 | 183.4 | 173.4 | 154.3 | 145.8 | 131.9 |
| | X | 220.6 | 183.0 | 174.5 | 153.5 | 145.7 | 132.3 |
| | RE/% | / | -17.05% | -20.88% | -30.42% | -33.94% | -40.04% |
| 5mmol/L | 1 | 221.3 | 180.5 | 172.1 | 151.3 | 143.6 | 130.6 |
| | 2 | 224.5 | 181.2 | 173.1 | 149.6 | 142.9 | 130.5 |
| | X | 222.9 | 180.9 | 172.6 | 150.5 | 143.3 | 130.6 |
| | RE/% | / | -18.86% | -22.57% | -32.50% | -35.73% | -41.43% |
| 20mmol/L | 1 | 222.3 | 182.5 | 172.1 | 158.4 | 151.9 | 129.6 |
| | 2 | 222.4 | 181.3 | 173.0 | 156.3 | 152.1 | 128.5 |
| | X | 222.4 | 181.9 | 172.6 | 157.4 | 152.0 | 129.1 |
| | RE/% | / | -18.19% | -22.40% | -29.23% | -31.64% | -41.96% |

| MAOS | etamsylate concentration | 0 mg/L | 50 mg/L | 100 mg/L | 150 mg/L | 200 mg/L | 250 mg/L |
|---|---|---|---|---|---|---|---|
| 0.3mmol/L | 1 | 228.5 | 185.9 | 175.7 | 152.6 | 145.6 | 132.3 |
| | 2 | 227.6 | 187.3 | 174.4 | 153.3 | 145.8 | 132.9 |
| | X | 228.1 | 186.6 | 175.1 | 153.0 | 145.7 | 132.6 |
| | RE/% | / | -18.18% | -23.24% | -32.93% | -36.11% | -41.85% |
| 5mmol/L | 1 | 222.6 | 184.2 | 175.3 | 154.3 | 142.6 | 134.6 |
| | 2 | 223.5 | 185.3 | 174.6 | 153.6 | 143.9 | 133.5 |
| | X | 223.1 | 184.8 | 175.0 | 154.0 | 143.3 | 134.1 |
| | RE/% | / | -17.17% | -21.56% | -30.98% | -35.78% | -39.90% |
| 20mmol/L | 1 | 220.8 | 180.7 | 171.2 | 149.4 | 142.9 | 130.4 |
| | 2 | 219.9 | 179.6 | 169.9 | 150.1 | 143.1 | 130.9 |
| | X | 220.4 | 180.2 | 170.6 | 149.8 | 143.0 | 130.7 |
| | RE/% | / | -18.24% | -22.60% | -32.04% | -35.10% | -40.71% |

| HDAOS | etamsylate concentration | 0 mg/L | 50 mg/L | 100 mg/L | 150 mg/L | 200 mg/L | 250 mg/L |
|---|---|---|---|---|---|---|---|
| 0.05mmol/L | 1 | 239.8 | 226.6 | 220.7 | 212.6 | 213.1 | 209.8 |
| | 2 | 237.2 | 225.1 | 217.3 | 213.7 | 211.4 | 208.7 |
| | X | 238.5 | 225.9 | 219.0 | 213.2 | 212.3 | 209.3 |
| | RE/% | | -5.30% | -8.18% | -10.63% | -11.01% | -12.26% |

(continued)

| HDAOS | etamsylate concentration | 0 mg/L | 50 mg/L | 100 mg/L | 150 mg/L | 200 mg/L | 250 mg/L |
|---|---|---|---|---|---|---|---|
| 0.1 mmol/L | 1 | 240.6 | 228.9 | 227.2 | 223.5 | 221.5 | 216.8 |
| | 2 | 235.4 | 231.5 | 225.8 | 223.4 | 220.1 | 214.5 |
| | X | 238.0 | 230.2 | 226.5 | 223.5 | 220.8 | 215.7 |
| | RE/% | | -3.28% | -4.83% | -6.11% | -7.23% | -9.39% |
| 0.3mmol/L | 1 | 241.8 | 232.0 | 229.3 | 228.3 | 224.3 | 220.5 |
| | 2 | 236.9 | 230.9 | 229.1 | 226.5 | 223.8 | 222.4 |
| | X | 239.4 | 231.5 | 229.2 | 227.4 | 224.1 | 221.5 |
| | RE/% | | -3.30% | -4.24% | -4.99% | -6.39% | -7.48% |
| 10mmol/L | 1 | 238.8 | 232.0 | 229.3 | 228.0 | 225.4 | 225.1 |
| | 2 | 236.9 | 230.9 | 229.1 | 227.6 | 225.6 | 224.5 |
| | X | 237.9 | 231.5 | 229.2 | 227.8 | 225.5 | 224.8 |
| | RE/% | | -2.69% | -3.64% | -4.23% | -5.19% | -5.49% |
| 20mmol/L | 1 | 236.3 | 225.6 | 222.1 | 218.5 | 216.1 | 215.5 |
| | 2 | 231.6 | 224.4 | 221.3 | 219.2 | 217.3 | 215.3 |
| | X | 234.0 | 225.0 | 221.7 | 218.9 | 216.7 | 215.4 |
| | RE/% | | -3.83% | -5.24% | -6.45% | -7.37% | -7.93% |
| 50mmol/L | 1 | 239.3 | 227.9 | 225.3 | 223.4 | 221.8 | 218.3 |
| | 2 | 234.6 | 228.9 | 224.1 | 224.1 | 221.3 | 218.6 |
| | X | 237.0 | 228.4 | 224.7 | 223.8 | 221.6 | 218.5 |
| | RE/% | | -3.61% | -5.17% | -5.57% | -6.50% | -7.81% |
| 60mmol/L | 1 | 240.3 | 225.6 | 220.5 | 218.6 | 216.2 | 216.5 |
| | 2 | 238.5 | 226.4 | 221.1 | 217.5 | 217.2 | 215.3 |
| | X | 239.4 | 226.0 | 220.8 | 218.1 | 216.7 | 215.9 |
| | RE/% | | -5.60% | -7.77% | -8.92% | -9.48% | -9.82% |
| 80mmol/L | 1 | 240.3 | 225.6 | 220.5 | 215.6 | 213.2 | 208.5 |
| | 2 | 238.5 | 226.4 | 221.1 | 216.5 | 211.2 | 209.6 |
| | X | 239.4 | 226.0 | 220.8 | 216.1 | 212.2 | 209.1 |
| | RE/% | | -5.60% | -7.77% | -9.75% | -11.36% | -12.68% |

| DAOS | etamsylate concentration | 0 mg/L | 50 mg/L | 100 mg/L | 150 mg/L | 200 mg/L | 250 mg/L |
|---|---|---|---|---|---|---|---|
| 0.05mmol/L | 1 | 234.4 | 218.6 | 211.8 | 208.2 | 205.5 | 203.2 |
| | 2 | 231.9 | 219.1 | 210.4 | 207.5 | 207.6 | 202.1 |
| | X | 233.2 | 218.9 | 211.1 | 207.9 | 206.6 | 202.7 |
| | RE/% | | -6.13% | -9.46% | -10.85% | -11.41% | -13.08% |
| 0.1mmol/L | 1 | 235.1 | 225.6 | 222.4 | 219.2 | 216.6 | 211.6 |
| | 2 | 230.2 | 224.4 | 223.2 | 218.9 | 215.2 | 210.7 |
| | X | 232.7 | 225.0 | 222.8 | 219.1 | 215.9 | 211.2 |
| | RE/% | | -3.29% | -4.23% | -5.85% | -7.20% | -9.24% |

(continued)

| DAOS | etamsylate concentration | 0 mg/L | 50 mg/L | 100 mg/L | 150 mg/L | 200 mg/L | 250 mg/L |
|---|---|---|---|---|---|---|---|
| 0.3mmol/L | 1 | 236.3 | 228.5 | 227.8 | 223.2 | 220.5 | 215.3 |
| | 2 | 235.2 | 226.3 | 225.1 | 222.3 | 221.2 | 218.6 |
| | X | 235.8 | 227.4 | 226.5 | 222.8 | 220.9 | 217.0 |
| | RE/% | | -3.54% | -3.94% | -5.51 % | -6.32% | -7.97% |
| 10mmol/L | 1 | 234.3 | 224.9 | 222.3 | 221.2 | 219.5 | 218.5 |
| | 2 | 229.6 | 223.9 | 223.1 | 220.5 | 220.9 | 217.9 |
| | X | 232.0 | 224.4 | 222.7 | 220.9 | 220.2 | 218.2 |
| | RE/% | | -3.26% | -3.99% | -4.79% | -5.07% | -5.93% |
| 20mmol/L | 1 | 237.3 | 227.9 | 225.3 | 220.5 | 219.8 | 216.5 |
| | 2 | 232.6 | 226.9 | 225.1 | 223.1 | 218.5 | 215.6 |
| | X | 234.95 | 227.4 | 225.2 | 221.8 | 219.15 | 216.05 |
| | RE/% | | -3.21% | -4.15% | -5.60% | -6.72% | -8.04% |
| 50mmol/L | 1 | 235.8 | 224.6 | 221.2 | 218.9 | 216.6 | 214.5 |
| | 2 | 231.3 | 223.5 | 220.5 | 217.8 | 216.3 | 215.1 |
| | X | 233.55 | 224.05 | 220.85 | 218.35 | 216.45 | 214.8 |
| | RE/% | | -4.07% | -5.44% | -6.51% | -7.32% | -8.03% |
| 60mmol/L | 1 | 234.1 | 219.3 | 217.5 | 214.3 | 212.2 | 211.6 |
| | 2 | 232.5 | 221.2 | 215.6 | 213.5 | 212.6 | 210.8 |
| | X | 233.3 | 220.25 | 216.55 | 213.9 | 212.4 | 211.2 |
| | RE/% | | -5.59% | -7.18% | -8.32% | -8.96% | -9.47% |
| 80mmol/L | 1 | 234.1 | 219.3 | 217.5 | 211.3 | 210.2 | 205.6 |
| | 2 | 232.5 | 221.2 | 215.6 | 210.5 | 208.6 | 204.8 |
| | X | 233.3 | 220.25 | 216.55 | 210.9 | 209.4 | 205.2 |
| | RE/% | | -5.59% | -7.18% | -9.60% | -10.24% | -12.04% |

From the above table, it can be seen that the HDAOS and DAOS in the wide concentration range of 0.1mmol/L-60mmol/L can resist the interference of no more than 250mg/Letamsylate, but only slightly higher than 10% when the etamsylate concentration is very high.

### 3. The concentration of creatininase

The different concentrations of creatininase, as shown in table 10, were prepared, and the remaining components were prepared in accordance with the formulation shown in example 1 except creatininase and chromogens type.

Freshly mixed serums are collected from healthy persons with no hemolytic, no turbid and no jaundice, and pure creatinine is added to the serums until its concentration is 200.0-250.0$\mu$mol/L (due to differences in the formulation, the measured value in different formulation will vary slightly, see in detail 0mg/L concentration of drugs sample values in various tables), which is served as basal serum. The etamsylate solution with the basic serum according to the ratio of 1:9 are prepared into 250mg/L drug concentration of interfered samples; control samples are prepared from saline and basal serum according to the ratio of 1:9; different concentration of interfered samples are prepared from 250mg/L drug concentration of interfered samples and control samples in different ratios. According to Example 3, the concentrations of creatinine in the control sample and the interfered samples of different concentration are measured, and the deviation from the control sample is calculated. The results are shown in Table 10.

**Table 10**. The effect of different concentrations of creatininase on anti-etamsylate interference

| HDAOS-different conconcentration creatininase | of | etamsylate | 0 mg/L 50 | mg/L 100 mg/L | 150 mg/L | 200 mg/L | 250 mg/L |
|---|---|---|---|---|---|---|---|
| R2: creatininase 30KU/L | 1 | 185.3 | 173.2 | 172.2 | 170.4 | 168.5 | 166.1 |
| | 2 | 184.6 | 172.8 | 171.5 | 170.1 | 168.4 | 167.0 |
| | X | 185.0 | 173.0 | 171.9 | 170.3 | 168.5 | 166.6 |
| | RE/% | | -6.46% | -7.08% | -7.95% | -8.92% | -9.95% |
| R2: creatininase 60KU/L | 1 | 188.2 | 175.7 | 175.1 | 173.6 | 172.5 | 170.1 |
| | 2 | 187.5 | 176.8 | 174.8 | 174.2 | 172.4 | 171.2 |
| | X | 187.9 | 176.3 | 175.0 | 173.9 | 172.5 | 170.7 |
| | RE/% | | -6.18% | -6.87% | -7.43% | -8.20% | -9.16% |
| R2: creatininase 105KU/L | 1 | 190.3 | 180.4 | 178.6 | 177.4 | 176.2 | 175.6 |
| | 2 | 191.2 | 180.6 | 178.9 | 178.3 | 176.8 | 175.1 |
| | X | 190.8 | 180.5 | 178.8 | 177.9 | 176.5 | 175.4 |
| | RE/% | | -5.37% | -6.29% | -6.76% | -7.47% | -8.07% |
| R2: creatininase 120KU/L | 1 | 192.4 | 186.8 | 186.1 | 183.2 | 182.8 | 180.9 |
| | 2 | 191.6 | 186.3 | 185.6 | 184.5 | 182.6 | 180.2 |
| | X | 192.0 | 186.6 | 185.9 | 183.9 | 182.7 | 180.6 |
| | RE/% | | -2.84% | -3.20% | -4.24% | -4.84% | -5.96% |
| R2:creatininase300KU/L | 1 | 189.4 | 184.3 | 182.6 | 181.5 | 179.5 | 178.5 |
| | 2 | 189.6 | 183.9 | 183.3 | 182.3 | 180.3 | 177.6 |
| | X | 189.5 | 184.1 | 183.0 | 181.9 | 179.9 | 178.1 |
| | RE/% | | -2.85% | -3.46% | -4.01% | -5.07% | -6.04% |
| R2: creatininase 500KU/L | 1 | 192.6 | 187.4 | 185.7 | 184.3 | 182.6 | 181.8 |
| | 2 | 193.2 | 187.2 | 185.2 | 183.9 | 183.4 | 180.9 |
| | X | 192.9 | 187.3 | 185.5 | 184.1 | 183.0 | 181.4 |
| | RE/% | | -2.90% | -3.86% | -4.56% | -5.13% | -5.99% |
| R2: creatininase 800KU/L | 1 | 191.4 | 183.4 | 181.6 | 180.1 | 178.5 | 176.4 |
| | 2 | 192.2 | 182.1 | 182.1 | 179.5 | 177.8 | 175.9 |
| | X | 191.8 | 182.8 | 181.9 | 179.8 | 178.2 | 176.2 |
| | RE/% | | -4.72% | -5.19% | -6.26% | -7.12% | -8.16% |
| R2: creatininase 1000KU/L | 1 | 194.4 | 182.1 | 180.2 | 178.1 | 176.2 | 175.3 |
| | 2 | 193.2 | 181.6 | 179.6 | 177.6 | 176.1 | 175.8 |
| | X | 193.8 | 181.9 | 179.9 | 177.9 | 176.2 | 175.6 |
| | RE/% | | -6.17% | -7.17% | -8.23% | -9.11% | -9.42% |
| | | | | | | | |
| DAOS-different conconcentration of creatininase | etamsylate | 0 mg/L | 50 mg/L | 100 mg/L | 150 mg/L | 200 mg/L | 250 mg/L |
| R2: creatininase | 1 | 166.5 | 155.3 | 153.6 | 152.9 | 151.3 | 148.9 |

(continued)

| DAOS-different conconcentration of creatininase | etamsylate | 0 mg/L | 50 mg/L | 100 mg/L | 150 mg/L | 200 mg/L | 250 mg/L |
|---|---|---|---|---|---|---|---|
| 30KU/L | 2 | 165.3 | 156.4 | 154.2 | 153.6 | 150.9 | 149.7 |
| | X | 165.9 | 155.9 | 153.9 | 153.3 | 151.1 | 149.3 |
| | RE/% | | -6.06% | -7.23% | -7.63% | -8.92% | -10.01% |
| R2: creatininase | 1 | 170.3 | 159.2 | 157.3 | 156.4 | 154.8 | 152.5 |
| 60KU/L | 2 | 168.9 | 160.1 | 158.1 | 156.1 | 155.2 | 153.2 |
| | X | 169.6 | 159.7 | 157.7 | 156.3 | 155.0 | 152.9 |
| | RE/% | | -5.87% | -7.02% | -7.87% | -8.61% | -9.88% |
| R2: creatininase | 1 | 175.2 | 168.2 | 164.5 | 163.2 | 162.8 | 161.4 |
| 105KU/L | 2 | 174.9 | 166.1 | 165.8 | 164.8 | 163.1 | 160.8 |
| | X | 175.1 | 167.2 | 165.2 | 164.0 | 163.0 | 161.1 |
| | RE/% | | -4.51% | -5.66% | -6.31% | -6.91% | -7.97% |
| R2: creatininase | 1 | 175.6 | 169.3 | 168.4 | 166.5 | 165.3 | 165.1 |
| 120KU/L | 2 | 176.2 | 169.9 | 167.8 | 167.8 | 166.2 | 165.6 |
| | X | 175.9 | 169.6 | 168.1 | 167.2 | 165.8 | 165.4 |
| | RE/% | | -3.58% | -4.43% | -4.97% | -5.77% | -6.00% |
| R2: creatininase 300KU/L | 1 | 175.4 | 169.2 | 167.5 | 165.6 | 164.3 | 164.1 |
| | 2 | 173.8 | 169.5 | 166.9 | 166.8 | 165.9 | 163.9 |
| | X | 174.6 | 169.35 | 167.2 | 166.2 | 165.1 | 164 |
| | RE/% | | -3.01% | -4.24% | -4.81% | -5.44% | -6.07% |
| R2: creatininase 500KU/L | 1 | 175.6 | 170.2 | 168.3 | 166.5 | 165.3 | 164.8 |
| | 2 | 174.9 | 169.2 | 167.4 | 166.9 | 165.8 | 164.9 |
| | X | 175.3 | 169.7 | 167.9 | 166.7 | 165.6 | 164.9 |
| | RE/% | | -3.17% | -4.22% | -4.88% | -5.53% | -5.93% |
| R2: creatininase 800KU/L | 1 | 170.5 | 162.4 | 161.5 | 160.1 | 158.7 | 157.8 |
| | 2 | 171.6 | 163.8 | 161.2 | 160.5 | 159.2 | 157.2 |
| | X | 171.1 | 163.1 | 161.4 | 160.3 | 159.0 | 157.5 |
| | RE/% | | -4.65% | -5.67% | -6.28% | -7.07% | -7.92% |
| R2: creatininase 1 0OOKU/L | 1 | 173.2 | 162.1 | 161.8 | 160.8 | 159.1 | 157.4 |
| | 2 | 174.1 | 163.7 | 162.0 | 160.4 | 158.4 | 156.9 |
| | X | 173.7 | 162.9 | 161.9 | 160.6 | 158.8 | 157.2 |
| | RE/% | | -6.19% | -6.77% | -7.52% | -8.58% | -9.50% |

As can be seen from the table above, all concentrations of creatininasecan achieve a deviation of less than 10% compared to the control sample. But in the range of 105-800KU / L, especially 120-500KU / L, the deviation is smaller.

## 4. The concentration of catalase

The different concentrations of catalase, as shown in table 11, were prepared, and the remaining components were prepared in accordance with the formulation shown in example 1 exceptcatalase and chromogens type.

Freshly mixed serums are collected from healthy persons with no hemolytic, no turbid and no jaundice, and pure creatinine is added to the serums until its concentration is 200.0-250.0μmol/L (due to differences in the formulation, the measured value in different formulation will vary slightly, see in detail 0mg/L concentration of drugs sample values in various tables), which is served as basal serum. The etamsylate solution with the basic serum according to the ratio of 1:9 are prepared into 250mg/L drug concentration of interfered samples; control samples are prepared from saline and basal serum according to the ratio of 1:9; different concentration of interfered samples are prepared from 250mg/L drug concentration of interfered samples and control samples in different ratios. According to Example 3, the concentrations of creatinine in the control sample and the interfered samples of different concentration are measured, and the deviation from the control sample is calculated. The results are shown in Table 11.

**Table 11.** Effects of different concentrations of catalase on the interference of anti-etamsylate interference

| HDAOS | etamsylate | 0 mg/L | 50 mg/L | 100 mg/L | 150 mg/L | 200 mg/L | 250 mg/L |
|---|---|---|---|---|---|---|---|
| R1: CAT 8KU/L | 1 | 225.6 | 209.6 | 207.5 | 206.3 | 204.8 | 204.2 |
|  | 2 | 226.8 | 208.6 | 207.8 | 205.9 | 205.5 | 203.9 |
|  | X | 226.2 | 209.1 | 207.7 | 206.1 | 205.2 | 204.1 |
|  | RE/% |  | -7.56% | -8.20% | -8.89% | -9.31 % | -9.79% |
| R1: CAT 20KU/L | 1 | 195.8 | 187.6 | 185.6 | 183.6 | 181.5 | 180.3 |
|  | 2 | 196.3 | 186.3 | 185.4 | 184.2 | 182.1 | 180.4 |
|  | X | 196.1 | 187.0 | 185.5 | 183.9 | 181.8 | 180.4 |
|  | RE/% |  | -4.64% | -5.38% | -6.20% | -7.27% | -8.01% |
| R1: CAT 50KU/L | 1 | 193.5 | 188.1 | 186.5 | 185.4 | 183.4 | 182.6 |
|  | 2 | 194.2 | 188.3 | 187.2 | 186.2 | 183.8 | 181.9 |
|  | X | 193.9 | 188.2 | 186.9 | 185.8 | 183.6 | 182.3 |
|  | RE/% |  | -2.91% | -3.61% | -4.15% | -5.29% | -5.98% |
| R1 :CAT 400KU/L | 1 | 192.4 | 188.4 | 185.2 | 184.1 | 182.3 | 181.3 |
|  | 2 | 193.6 | 187.9 | 185.9 | 183.5 | 182.1 | 181.4 |
|  | X | 193.0 | 188.2 | 185.6 | 183.8 | 182.2 | 181.4 |
|  | RE/% |  | -2.51% | -3.86% | -4.77% | -5.60% | -6.04% |
| R1 :CAT 600KU/L | 1 | 195.4 | 189.5 | 188.2 | 186.1 | 185.5 | 183.7 |
|  | 2 | 195.6 | 190.2 | 187.4 | 186.5 | 184.4 | 183.9 |
|  | X | 195.5 | 189.85 | 187.8 | 186.3 | 184.95 | 183.8 |
|  | RE/% |  | -2.89% | -3.94% | -4.71% | -5.40% | -5.98% |
| R1: CAT 1 0OOKU/L | 1 | 193.4 | 184.4 | 182.7 | 181.6 | 180.4 | 178.7 |
|  | 2 | 194.2 | 184.2 | 183.2 | 180.8 | 179.6 | 177.9 |
|  | X | 193.8 | 184.3 | 182.95 | 181.2 | 180 | 178.3 |
|  | RE/% |  | -4.90% | -5.60% | -6.50% | -7.12% | -8.00% |
| R1 :CAT 1500KU/L | 1 | 190.1 | 180.1 | 179.2 | 176.3 | 174.3 | 173.1 |
|  | 2 | 191.2 | 181.3 | 178.4 | 177.2 | 175.1 | 171.2 |
|  | X | 190.65 | 180.7 | 178.8 | 176.75 | 174.7 | 172.15 |
|  | RE/% |  | -5.22% | -6.22% | -7.29% | -8.37% | -9.70% |

(continued)

| DAOS | etamsylate | 0 mg/L | 50 mg/L | 100 mg/L | 150 mg/L | 200 mg/L | 250 mg/L |
|---|---|---|---|---|---|---|---|
| R1:CAT 10KU/L | 1 | 223.6 | 208.1 | 206.2 | 204.3 | 203.6 | 202.6 |
| | 2 | 224.7 | 207.5 | 205.4 | 204.8 | 203.4 | 202.1 |
| | X | 224.2 | 207.8 | 205.8 | 204.6 | 203.5 | 202.4 |
| | RE/% | | -7.29% | -8.19% | -8.74% | -9.21% | -9.73% |
| R1: CAT 20KU/L | 1 | 193.4 | 186.4 | 183.4 | 181.5 | 180.1 | 179.3 |
| | 2 | 195.3 | 185.1 | 184.2 | 182.3 | 180.6 | 178.4 |
| | X | 194.4 | 185.8 | 183.8 | 181.9 | 180.4 | 178.9 |
| | RE/% | | -4.43% | -5.43% | -6.41% | -7.20% | -7.98% |
| R1 :CAT 50KU/L | 1 | 193.4 | 185.6 | 185.1 | 183.6 | 182.5 | 181.8 |
| | 2 | 193.3 | 186.1 | 184.3 | 183.1 | 182.3 | 182.0 |
| | X | 193.4 | 185.9 | 184.7 | 183.4 | 182.4 | 181.9 |
| | RE/% | | -3.88% | -4.47% | -5.17% | -5.66% | -5.92% |
| R1 :CAT 400KU/L | 1 | 194.4 | 187.3 | 185.2 | 185.1 | 184.1 | 182.8 |
| | 2 | 195.1 | 188.5 | 186.4 | 184.5 | 183.8 | 183.1 |
| | X | 194.8 | 187.9 | 185.8 | 184.8 | 184.0 | 183.0 |
| | RE/% | | -3.52% | -4.60% | -5.11% | -5.55% | -6.06% |
| R1 :CAT 600KU/L | 1 | 195.1 | 189.5 | 187.2 | 185.2 | 184.5 | 183.5 |
| | 2 | 194.8 | 188.6 | 186.4 | 185.1 | 183.9 | 182.7 |
| | X | 195.0 | 189.1 | 186.8 | 185.2 | 184.2 | 183.1 |
| | RE/% | | -3.03% | -4.18% | -5.03% | -5.51% | -6.08% |
| R1: CAT 1000KU/L | 1 | 190.4 | 183.4 | 180.7 | 177.1 | 176.9 | 175.5 |
| | 2 | 191.2 | 184.1 | 180.2 | 178.3 | 176.5 | 175.6 |
| | X | 190.8 | 183.75 | 180.45 | 177.7 | 176.7 | 175.55 |
| | RE/% | | -3.69% | -5.42% | -6.87% | -7.39% | -7.99% |
| R1 :CAT 1500KU/L | 1 | 189.0 | 180.3 | 178.3 | 176.2 | 174.3 | 172.3 |
| | 2 | 190.1 | 179.5 | 177.6 | 175.8 | 174.1 | 171.5 |
| | X | 189.55 | 179.9 | 177.95 | 176 | 174.2 | 171.9 |
| | RE/% | | -5.09% | -6.12% | -7.15% | -8.10% | -9.31% |

As can be seen from the table above, all concentrations of catalase can achieve a deviation of less than 10% compared to the control sample. But in the range of 50-600KU/L, the deviation is smaller.

### 5. The concentration of sarcosine oxidase, creatinase and peroxidase

The different concentrations of sarcosine oxidase, creatinase and peroxidase, as shown in table 12, were prepared, and the remaining components were prepared in accordance with the formulation shown in example 1 except sarcosineoxidase, keratinase, peroxidase and chromogens type.

Freshly mixed Serums are collected from healthy persons with no hemolytic, no turbid and no jaundice, and pure creatinine is added to the serums until its concentration is 200.0-250.0$\mu$mol/L (due to differences in the formulation, the measured value in different formulation will vary slightly, see in detail 0mg/L concentration of drugs sample values in various tables), which is served as basal serum. The etamsylate solution with the basic serum according to the ratio of 1:9 are prepared into 250mg/L drug concentration of interfered samples; control samples are prepared from saline and basal serum according to the ratio of 1:9; different concentration of interfered samples are prepared from 250mg/L drug concentration of interfered samples and control samples in different ratios. According to Example 3, the concentrations of creatinine in the control sample and the interfered samples of different concentration are measured, and the deviation from the control sample is calculated. The results are shown in Table 12.

**Table 12.**

| HDAOS | etamsylate concentration | 0 mg/L | 50 mg/L | 100 mg/L | 150 mg/L | 200 mg/L | 250 mg/L |
|---|---|---|---|---|---|---|---|
| sarcosine oxidase: 0.3KU/L | 1 | 236.4 | 229.6 | 227.4 | 223.3 | 221.2 | 220.5 |
|  | 2 | 237.3 | 228.4 | 226.5 | 224.4 | 222.5 | 220.4 |
|  | X | 236.9 | 229.0 | 227.0 | 223.9 | 221.9 | 220.5 |
|  | RE/% | / | -3.31% | -4.18% | -5.49% | -6.33% | -6.92% |
| sarcosine oxidase: 0.5KU/L | 1 | 240.2 | 232.6 | 230.6 | 228.6 | 227.8 | 224.8 |
|  | 2 | 239.5 | 232.3 | 231.2 | 229.3 | 226.9 | 225.9 |
|  | X | 239.9 | 232.5 | 230.9 | 229.0 | 227.4 | 225.4 |
|  | RE/% | / | -3.09% | -3.73% | -4.54% | -5.21% | -6.05% |
| sarcosine oxidase: 2KU/L | 1 | 238.5 | 231.8 | 229.6 | 227.4 | 225.4 | 223.6 |
|  | 2 | 237.6 | 232.6 | 228.7 | 226.5 | 226.1 | 224.5 |
|  | X | 238.1 | 232.2 | 229.2 | 227.0 | 225.8 | 224.1 |
|  | RE/% | / | -2.46% | -3.74% | -4.66% | -5.17% | -5.88% |
| sarcosine oxidase: 50KU/L | 1 | 241.3 | 233.5 | 231.6 | 230.4 | 228.6 | 225.3 |
|  | 2 | 240.5 | 232.1 | 231.1 | 229.5 | 228.1 | 226.4 |
|  | X | 240.9 | 232.8 | 231.4 | 230.0 | 228.4 | 225.9 |
|  | RE/% | / | -3.36% | -3.96% | -4.55% | -5.21% | -6.25% |
| sarcosine oxidase: 100KU/L | 1 | 235.2 | 229.6 | 227.5 | 225.8 | 223.6 | 221.6 |
|  | 2 | 236.4 | 230.5 | 227.1 | 226.1 | 224.2 | 222.3 |
|  | X | 235.8 | 230.1 | 227.3 | 226.0 | 223.9 | 222.0 |
|  | RE/% | / | -2.44% | -3.60% | -4.18% | -5.05% | -5.87% |
| sarcosine oxidase:150KU/L | 1 | 238.4 | 230.3 | 228.9 | 226.4 | 225.9 | 223.4 |
|  | 2 | 238.1 | 231.5 | 229.5 | 227.1 | 226.3 | 224.3 |
|  | X | 238.3 | 230.9 | 229.2 | 226.8 | 226.1 | 223.9 |
|  | RE/% | / | -3.08% | -3.80% | -4.83% | -5.10% | -6.04% |
| sarcosine oxidase:200KU/L | 1 | 236.7 | 229.4 | 226.3 | 223.4 | 222.9 | 219.1 |
|  | 2 | 235.8 | 228.6 | 225.7 | 224.1 | 222.5 | 220.3 |
|  | X | 236.3 | 229.0 | 226.0 | 223.8 | 222.7 | 219.7 |
|  | RE/% | / | -3.07% | -4.34% | -5.29% | -5.74% | -7.01% |

| DAOS | etamsylate concentration | 0 mg/L | 50 mg/L | 100 mg/L | 150 mg/L | 200 mg/L | 250 mg/L |
|---|---|---|---|---|---|---|---|
| sarcosine oxidase:0.3KU/L | 1 | 234.1 | 228.1 | 226.1 | 222.6 | 220.7 | 217.5 |
|  | 2 | 233.2 | 227.2 | 225.3 | 222.5 | 220.6 | 216.9 |
|  | X | 233.7 | 227.7 | 225.7 | 222.6 | 220.7 | 217.2 |

(continued)

| DAOS | etamsylate concentration | 0 mg/L | 50 mg/L | 100 mg/L | 150 mg/L | 200 mg/L | 250 mg/L |
|---|---|---|---|---|---|---|---|
| | RE/% | / | -2.57% | -3.40% | -4.75% | -5.56% | -7.04% |
| sarcosine | 1 | 235.2 | 230.6 | 228.5 | 226.6 | 223.8 | 221.5 |
| oxidase:0.5KU/L | 2 | 236.5 | 230.3 | 227.2 | 225.3 | 224.5 | 221.2 |
| | X | 235.9 | 230.5 | 227.9 | 226.0 | 224.2 | 221.4 |
| | RE/% | / | -2.29% | -3.39% | -4.20% | -4.96% | -6.15% |
| sarcosine | 1 | 230.4 | 225.0 | 224.6 | 221.8 | 220.4 | 217.3 |
| oxidase:2KU/L | 2 | 232.1 | 224.3 | 223.8 | 222.1 | 221.1 | 216.8 |
| | X | 231.3 | 224.7 | 224.2 | 222.0 | 220.8 | 217.1 |
| | RE/% | / | -2.85% | -3.05% | -4.02% | -4.54% | -6.14% |
| sarcosine | 1 | 231.3 | 223.9 | 221.8 | 218.6 | 217.6 | 216.8 |
| oxidase:50KU/L | 2 | 229.5 | 225.1 | 221.3 | 219.4 | 218.4 | 216.4 |
| | X | 230.4 | 224.5 | 221.6 | 219.0 | 218.0 | 216.6 |
| | RE/% | / | -2.56% | -3.84% | -4.95% | -5.38% | -5.99% |
| sarcosine | 1 | 233.2 | 225.4 | 223.2 | 222.3 | 219.5 | 218.7 |
| oxidase:100KU/L | 2 | 232.4 | 225.9 | 223.8 | 221.9 | 220.6 | 218.9 |
| | X | 232.8 | 225.7 | 223.5 | 222.1 | 220.1 | 218.8 |
| | RE/% | / | -3.07% | -3.99% | -4.60% | -5.48% | -6.01% |
| sarcosine | 1 | 234.4 | 228.4 | 226.4 | 223.4 | 221.6 | 221.3 |
| oxidase:150KU/L | 2 | 235.1 | 227.6 | 225.9 | 224.2 | 222.1 | 219.8 |
| | X | 234.8 | 228.0 | 226.2 | 223.8 | 221.9 | 220.6 |
| | RE/% | / | -2.88% | -3.66% | -4.66% | -5.50% | -6.05% |
| sarcosine | 1 | 231.6 | 224.6 | 223.1 | 221.1 | 217.3 | 215.9 |
| oxidase:200KU/L | 2 | 232.2 | 225.1 | 222.0 | 220.5 | 218.4 | 216.3 |
| | X | 231.9 | 224.9 | 222.6 | 220.8 | 217.9 | 216.1 |
| | RE/% | / | -3.04% | -4.03% | -4.79% | -6.06% | -6.81% |
| | | | | | | | |
| HDAOS | etamsylate concentration | 0 mg/L | 50 mg/L | 100 mg/L | 150 mg/L | 200 mg/L | 250 mg/L |
| creatinase: | 1 | 234.6 | 229.1 | 224.3 | 221.6 | 220.1 | 217.5 |
| 0.5KU/L | 2 | 233.1 | 228.4 | 225.3 | 220.9 | 219.3 | 216.6 |
| | X | 233.9 | 228.8 | 224.8 | 221.3 | 219.7 | 217.1 |
| | RE/% | / | -2.18% | -3.87% | -5.39% | -6.05% | -7.18% |
| creatinase: | 1 | 235.6 | 229.6 | 227.6 | 225.1 | 223.1 | 221.5 |
| 1KU/L | 2 | 236.1 | 230.1 | 226.8 | 224.9 | 222.5 | 221.9 |
| | X | 235.9 | 229.9 | 227.2 | 225.0 | 222.8 | 221.7 |
| | RE/% | / | -2.54% | -3.67% | -4.60% | -5.53% | -6.00% |
| | 1 | 234.5 | 228.4 | 226.1 | 223.4 | 221.5 | 221.2 |

(continued)

| HDAOS | etamsylate concentration | 0 mg/L | 50 mg/L | 100 mg/L | 150 mg/L | 200 mg/L | 250 mg/L |
|---|---|---|---|---|---|---|---|
| creatinase: 5KU/L | 2 | 235.1 | 228.1 | 225.8 | 224.1 | 222.3 | 220.1 |
| | X | 234.8 | 228.3 | 226.0 | 223.8 | 221.9 | 220.7 |
| | RE/% | / | -2.79% | -3.77% | -4.71% | -5.49% | -6.03% |
| creatinase: 75KU/L | 1 | 237.3 | 231.4 | 228.1 | 226.4 | 223.4 | 222.2 |
| | 2 | 236.6 | 230.5 | 227.6 | 225.3 | 224.1 | 222.8 |
| | X | 237.0 | 231.0 | 227.9 | 225.9 | 223.8 | 222.5 |
| | RE/% | / | -2.53% | -3.84% | -4.68% | -5.57% | -6.10% |
| creatinase: 150KU/L | 1 | 235.8 | 229.2 | 226.5 | 224.8 | 222.6 | 221.6 |
| | 2 | 235.4 | 228.1 | 227.0 | 225.1 | 223.1 | 221.8 |
| | X | 235.6 | 228.7 | 226.8 | 225.0 | 222.9 | 221.7 |
| | RE/% | / | -2.95% | -3.76% | -4.52% | -5.41% | -5.90% |
| creatinase: 300KU/L | 1 | 234.2 | 227.6 | 225.6 | 224.1 | 222.1 | 220.3 |
| | 2 | 234.6 | 228.5 | 226.3 | 223.3 | 221.6 | 220.6 |
| | X | 234.4 | 228.1 | 226.0 | 223.7 | 221.9 | 220.5 |
| | RE/% | / | -2.71% | -3.60% | -4.56% | -5.35% | -5.95% |
| creatinase: 400KU/L | 1 | 233.8 | 226.8 | 224.3 | 222.1 | 219.8 | 217.6 |
| | 2 | 234.1 | 227.2 | 225.1 | 222.3 | 220.5 | 218.0 |
| | X | 234.0 | 227.0 | 224.7 | 222.2 | 220.2 | 217.8 |
| | RE/% | / | -2.97% | -3.95% | -5.02% | -5.90% | -6.90% |
| | | | | | | | |
| DAOS | etamsylate concentration | 0 mg/L | 50 mg/L | 100 mg/L | 150 mg/L | 200 mg/L | 250 mg/L |
| creatinase: 0.5KU/L | 1 | 233.1 | 226.6 | 224.2 | 221.1 | 218.9 | 216.4 |
| | 2 | 232.8 | 225.4 | 223.9 | 221.8 | 219.0 | 216.2 |
| | X | 233.0 | 226.0 | 224.1 | 221.5 | 219.0 | 216.3 |
| | RE/% | / | -2.98% | -3.82% | -4.94% | -6.01% | -7.15% |
| creatinase: 1KU/L | 1 | 234.2 | 228.1 | 225.6 | 223.4 | 221.9 | 220.5 |
| | 2 | 233.8 | 227.4 | 226.3 | 222.8 | 221.4 | 219.6 |
| | X | 234.0 | 227.8 | 226.0 | 223.1 | 221.7 | 220.1 |
| | RE/% | / | -2.67% | -3.44% | -4.66% | -5.28% | -5.96% |
| creatinase: 5KU/L | 1 | 232.3 | 225.8 | 224.7 | 222.5 | 220.6 | 219.1 |
| | 2 | 233.1 | 226.3 | 223.9 | 221.1 | 221.3 | 218.8 |
| | X | 232.7 | 226.1 | 224.3 | 221.8 | 221.0 | 219.0 |
| | RE/% | / | -2.86% | -3.61% | -4.68% | -5.05% | -5.91% |

(continued)

| DAOS | etamsylate concentration | 0 mg/L | 50 mg/L | 100 mg/L | 150 mg/L | 200 mg/L | 250 mg/L |
|---|---|---|---|---|---|---|---|
| creatinase: 75KU/L | 1 | 231.4 | 223.6 | 221.8 | 220.3 | 218.4 | 217.5 |
| | 2 | 230.6 | 224.9 | 222.3 | 219.8 | 219.6 | 216.9 |
| | X | 231.0 | 224.3 | 222.1 | 220.1 | 219.0 | 217.2 |
| | RE/% | / | -2.92% | -3.87% | -4.74% | -5.19% | -5.97% |
| creatinase: 150KU/L | 1 | 232.5 | 226.6 | 224.1 | 222.4 | 219.8 | 219.1 |
| | 2 | 232.7 | 225.7 | 223.5 | 221.5 | 220.4 | 218.2 |
| | X | 232.6 | 226.2 | 223.8 | 222.0 | 220.1 | 218.7 |
| | RE/% | / | -2.77% | -3.78% | -4.58% | -5.37% | -6.00% |
| creatinase: 300KU/L | 1 | 233.4 | 226.8 | 224.6 | 222.4 | 219.8 | 218.6 |
| | 2 | 232.1 | 227.1 | 225.1 | 221.9 | 220.3 | 219.0 |
| | X | 232.8 | 227.0 | 224.9 | 222.2 | 220.1 | 218.8 |
| | RE/% | / | -2.49% | -3.39% | -4.55% | -5.46% | -5.99% |
| creatinase: 400KU/L | 1 | 233.4 | 225.6 | 224.3 | 221.3 | 218.6 | 216.5 |
| | 2 | 232.1 | 226.1 | 223.9 | 220.8 | 219.3 | 217.1 |
| | X | 232.8 | 225.9 | 224.1 | 221.1 | 219.0 | 216.8 |
| | RE/% | / | -2.96% | -3.72% | -5.03% | -5.93% | -6.85% |
| HDAOS | etamsylate concentration | 0 mg/L | 50 mg/L | 100 mg/L | 150 mg/L | 200 mg/L | 250 mg/L |
| peroxidase: 0.5KU/L | 1 | 219.3 | 213.1 | 212.1 | 210.1 | 207.3 | 204.8 |
| | 2 | 220.1 | 214.1 | 211.3 | 209.5 | 206.8 | 203.6 |
| | X | 219.7 | 213.6 | 211.7 | 209.8 | 207.1 | 204.2 |
| | RE/% | / | -2.78% | -3.64% | -4.51% | -5.76% | -7.06% |
| peroxidase: 1KU/L | 1 | 220.6 | 214.6 | 213.5 | 210.5 | 208.6 | 207.5 |
| | 2 | 221.1 | 215.4 | 212.4 | 210.8 | 208.9 | 208.1 |
| | X | 220.9 | 215.0 | 213.0 | 210.7 | 208.8 | 207.8 |
| | RE/% | / | -2.65% | -3.58% | -4.62% | -5.48% | -5.91% |
| peroxidase: 25KU/L | 1 | 221.5 | 216.4 | 214.6 | 212.1 | 209.4 | 207.5 |
| | 2 | 221.3 | 215.2 | 213.8 | 211.5 | 210.1 | 208.6 |
| | X | 221.4 | 215.8 | 214.2 | 211.8 | 209.8 | 208.1 |
| | RE/% | / | -2.53% | -3.25% | -4.34% | -5.26% | -6.03% |
| peroxidase: 50KU/L | 1 | 220.3 | 214.8 | 213.2 | 210.4 | 209.4 | 206.8 |
| | 2 | 220.1 | 215.1 | 212.8 | 211.3 | 208.7 | 207.4 |
| | X | 220.2 | 215.0 | 213.0 | 210.9 | 209.1 | 207.1 |
| | RE/% | / | -2.38% | -3.27% | -4.25% | -5.06% | -5.95% |

(continued)

| HDAOS | etamsylate concentration | 0 mg/L | 50 mg/L | 100 mg/L | 150 mg/L | 200 mg/L | 250 mg/L |
|---|---|---|---|---|---|---|---|
| peroxidase: 100KU/L | 1 | 219.5 | 213.6 | 211.4 | 210.1 | 207.6 | 205.7 |
| | 2 | 218.8 | 214.1 | 212.0 | 209.4 | 208.1 | 206.2 |
| | X | 219.2 | 213.9 | 211.7 | 209.8 | 207.9 | 206.0 |
| | RE/% | / | -2.42% | -3.40% | -4.29% | -5.16% | -6.02% |
| peroxidase: 200KU/L | 1 | 218.5 | 212.1 | 209.4 | 207.3 | 204.3 | 202.3 |
| | 2 | 217.3 | 211.6 | 210.1 | 206.8 | 205.1 | 203.1 |
| | X | 217.9 | 211.9 | 209.8 | 207.1 | 204.7 | 202.7 |
| | RE/% | / | -2.78% | -3.74% | -4.98% | -6.06% | -6.98% |
| | | | | | | | |
| DAOS | etamsylate concentration | 0 mg/L | 50 mg/L | 100 mg/L | 150 mg/L | 200 mg/L | 250 mg/L |
| peroxidase: 0.5KU/L | 1 | 216.1 | 210.3 | 207.3 | 205.1 | 203.1 | 200.1 |
| | 2 | 215.8 | 209.6 | 206.8 | 204.3 | 202.6 | 199.6 |
| | X | 216.0 | 210.0 | 207.1 | 204.7 | 202.9 | 199.9 |
| | RE/% | / | -2.78% | -4.12% | -5.21% | -6.07% | -7.46% |
| peroxidase: 1KU/L | 1 | 218.1 | 212.3 | 210.3 | 207.5 | 205.6 | 204.3 |
| | 2 | 217.8 | 213.2 | 209.5 | 206.8 | 206.1 | 205.2 |
| | X | 218.0 | 212.8 | 209.9 | 207.2 | 205.9 | 204.8 |
| | RE/% | / | -2.39% | -3.69% | -4.96% | -5.55% | -6.06% |
| peroxidase: 25KU/L | 1 | 217.4 | 212.2 | 209.8 | 206.5 | 205.8 | 204.5 |
| | 2 | 216.9 | 211.5 | 209.3 | 206.4 | 205.2 | 203.8 |
| | X | 217.2 | 211.9 | 209.6 | 206.5 | 205.5 | 204.2 |
| | RE/% | / | -2.44% | -3.50% | -4.93% | -5.36% | -5.99% |
| peroxidase: 50KU/L | 1 | 218.1 | 214.2 | 211.1 | 209.4 | 206.7 | 205.3 |
| | 2 | 218.6 | 213.5 | 212.1 | 208.5 | 207.1 | 206.2 |
| | X | 218.4 | 213.9 | 211.6 | 209.0 | 206.9 | 205.8 |
| | RE/% | / | -2.06% | -3.09% | -4.31% | -5.24% | -5.77% |
| peroxidase: 100KU/L | 1 | 217.5 | 212.2 | 210.4 | 208.4 | 206.5 | 204.7 |
| | 2 | 218.1 | 213.2 | 209.6 | 208.9 | 205.6 | 205.1 |
| | X | 217.8 | 212.7 | 210.0 | 208.7 | 206.1 | 204.9 |
| | RE/% | / | -2.34% | -3.58% | -4.20% | -5.39% | -5.92% |
| peroxidase: 200KU/L | 1 | 220.5 | 213.4 | 211.5 | 208.6 | 206.1 | 203.2 |
| | 2 | 219.1 | 214.3 | 210.6 | 207.7 | 205.3 | 204.1 |
| | X | 219.8 | 213.9 | 211.1 | 208.2 | 205.7 | 203.7 |
| | RE/% | / | -2.71% | -3.98% | -5.30% | -6.41% | -7.35% |

It can be seen from the above that the concentration of sarcosine oxidase, creatinase and peroxidase has no effect on the resistance of etamsylate interference in the detection of creatinine.

**6. Comparison with commercially available kits**

**6.1 Comparison with commercially available kit 1**

**6.1.1** Main components of commercially available kit 1 The first reagent set contains 2000U/L creatinase, 6000U/L sarcosine oxidase, 5000U/L peroxidase and 0.4 mmol/L N-ethyl-N-(3-sulfopropyl)-3-Methylaniline (TOPS); the second reagent set contains 25000 U/L creatininase and 1.0 mmol/L 4-AAP.

6.1.2 Results

The detection was carried out according to the method described above. The results are shown in Table 13 below:

**Table 13:** Effects of commercially available kit 1 on the interference of anti-etamsylate interference

| etamsylate concentration | 0 mg/L | 50 mg/L | 100 mg/L | 150 mg/L | 200 mg/L | 250 mg/L |
|---|---|---|---|---|---|---|
| 1 | 143.3 | 92.9 | 65.3 | 52.8 | 45.8 | 40.4 |
| 2 | 143.3 | 93.2 | 68.5 | 58.5 | 51.9 | 46.5 |
| M | 143.3 | 93.1 | 66.9 | 55.7 | 48.9 | 43.5 |
| RE/% | / | -35.07% | -53.31% | -61.17% | -65.91% | -69.68% |
| Interference or not | / | Yes | Yes | Yes | Yes | Yes |

It can be seen that the commercially available kit 1 is not resistant to the interference of etamsylate in the detection of creatinine.

**6.2 Comparison with commercially available kit 2**

**6.2.1** Main components of commercially available kit 2

The first Reagent set contains 75IU/ml creatinedehydratase, 11 IU/ml sarcosine oxidase, 0.3 mmol/L N-ethyl-N- (2-hydroxy-3-sulfopropyl) -3,5- dimethoxy-4-fluoroaniline sodium salt (F-DAOS); the second reagent setcontains 330 IU/ml creatininase and 4.9 mmol/L 4-AAP.

**6.2.2** Results

The test was carried out according to the method described above. The results are shown in Table 14 below:

**Table 14:** Effects of commercially available kit 2 on the interference of anti-etamsylate interference.

| etamsylate | 0 mg/L | 50 mg/L | 100 mg/L | 150 mg/L | 200 mg/L | 250 mg/L |
|---|---|---|---|---|---|---|
| 1 | 177.6 | 120.4 | 93.8 | 83.9 | 74.0 | 69.8 |
| 2 | 177.5 | 119.7 | 93.9 | 82.7 | 75.6 | 69.5 |
| M | 177.6 | 120.1 | 93.9 | 83.3 | 74.8 | 69.7 |
| RE/% | / | -32.39% | -47.14% | -53.08% | -57.87% | -60.77% |
| Interference or not | / | Yes | Yes | Yes | Yes | Yes |

It can be seen that the commercially available kit 2 is not resistant to the interference of etamsylate in the detection of creatinine.

**6.3 Comparison with other commercially available kits**

The use of commercially available kits 3, 4 and 5 for the detection of etamsylate interference is shown in Table 15. Among them, the main components of the commercially available kit 3 (Shanghai Kehua Bio-engineering Co., Lt) are: the first reagent set: creatine amidino hydrolase 40KU/L, sarcosine oxidase 7KU/L, ascorbate oxidase 2KU/L, catalase100KU/L, ESPMT 0.47mmol/L; the second reagent set: Creatinineamidinohydrolase 400KU/L, peroxidase 50KU/L, 4-AAP 2.95mmol/L. The main components of the commercially available kit 4 (Beijing Strong Biotechnologies, Inc) are: the first reagent set: creatinase 60 U / ml, sarcosine oxidase 15 U / ml, EMSE1.4 mmol/1; the second reagent set: peroxidase 30 U / ml, creatininase 310 U / ml, 4-AAP 2.5 mmol / L. The main components of the commercially available kit 5 (Roche Diagnostics) are the first reagent set: creatinase 332 μkat / L, sarcosine oxidase 132 μkat / L, catalase 1.67 μkat / L; HTIB 1.2 g / L, ascorbic acid oxidase 33μkat / L; the second reagent set: creatininase 498μkat / L, peroxidase 16.6μkat / L, 4-AAP 0.5g / L.

**Table 15:** Effects of commercially available kits 3-5 on the interference of anti-etamsylate interference – m : Measured; it : interference

| | etamsylate concentration | 0 mg/L | 25 mg/L | 50 mg/L | 100 mg/L | 150 mg/L | 200 mg/L | 250 mg/L |
|---|---|---|---|---|---|---|---|---|
| commercially available kit 3 | 1 | 142.5 | 128.9 | 116.8 | 99.7 | 86.0 | 75.4 | 67.7 |
| | 2 | 142.5 | 129.3 | 117.9 | 100.3 | 85.8 | 77.7 | 67.9 |
| | 3 | 142.5 | 128.3 | 117.2 | 101.2 | 86.4 | 76.5 | 68.2 |
| | M Average value | 142.5 | 128.8 | 117.3 | 100.4 | 86.1 | 76.5 | 67.9 |
| | it degree/ % | | -9.59 | -17.68 | -29.54 | -39.60 | -46.29 | -47.27 |
| | it or not | | NO | Yes | Yes | Yes | Yes | Yes |
| commercially available kit 4 | 1 | 135.9 | 123.9 | 114.6 | 98.0 | 85.4 | 78.5 | 70.2 |
| | 2 | 135.9 | 124.8 | 114.8 | 99.7 | 87.7 | 77.0 | 67.8 |
| | 3 | 135.9 | 125.1 | 115.0 | 99.2 | 87.1 | 78.2 | 70.6 |
| | M Average value | 135.9 | 124.6 | 114.8 | 99.0 | 86.7 | 77.9 | 69.5 |
| | it degree /% | | -8.31 | -15.53 | -27.18 | -36.18 | -42.68 | -44.19 |
| | it or not | | NO | Yes | Yes | Yes | Yes | Yes |
| commercially available kit 5 | 1 | 147.2 | 126.1 | 108.4 | 84.9 | 70.6 | 61.6 | 53.9 |
| | 2 | 147.2 | 124.6 | 108.2 | 86.1 | 70.1 | 60.9 | 53.8 |
| | 3 | 147.2 | 124.5 | 107.5 | 85.2 | 70.0 | 61.7 | 54.7 |
| | M Average value | 147.2 | 125.1 | 108.0 | 85.4 | 70.2 | 61.4 | 54.1 |
| | it degree/% | | -15.01% | -26.58 | -41.96 | -52.27 | -58.26 | -56.72 |
| | it or not | | Yes | Yes | Yes | Yes | Yes | Yes |

**Example 7**: **Discussion on the interference condition of Calcium Dobesilate**

According to the method of example 6, the interference conditions of Calcium Dobesilate was discussed. It was found that the interference resistance of calcium dobesilate could be achieved under the condition that the concentration and the components of etamsylate interference could be resisted. The data are shown in table 16:

**Table 16** Discussion on the interference condition of Calcium Dobesilate

| HDAOS | Calcium Dobesilate | 0 mg/L | 20 mg/L | 40 mg/L | 60 mg/L | 80 mg/L | 100 mg/L |
|---|---|---|---|---|---|---|---|
| 4-AAP:0.25 | 1 | 208.6 | 203.6 | 202.6 | 199.5 | 195.6 | 193.1 |
| | 2 | 209.3 | 204.3 | 201.2 | 198.3 | 194.3 | 192.6 |
| | X | 209.0 | 204.0 | 201.9 | 198.9 | 195.0 | 192.9 |
| | RE/% | | -2.39% | -3.37% | -4.81% | -6.70% | -7.71% |
| 4-AAP:0.5 | 1 | 208.5 | 204.4 | 203.1 | 201.1 | 200.5 | 199.2 |
| | 2 | 206.3 | 205.1 | 202.5 | 201.6 | 199.8 | 198.7 |
| | X | 207.4 | 204.8 | 202.8 | 201.4 | 200.2 | 199.0 |
| | RE/% | | -1.28% | -2.22% | -2.92% | -3.50% | -4.07% |

(continued)

| HDAOS | Calcium Dobesilate | 0 mg/L | 20 mg/L | 40 mg/L | 60 mg/L | 80 mg/L | 100 mg/L |
|---|---|---|---|---|---|---|---|
| 4-AAP:1.0 | 1 | 209.2 | 202.5 | 201.3 | 198.6 | 197.1 | 195.3 |
| | 2 | 204.7 | 203.6 | 200.5 | 199.1 | 196.8 | 194.8 |
| | X | 207.0 | 203.1 | 200.9 | 198.9 | 197.0 | 195.1 |
| | RE/% | | -1.88% | -2.92% | -3.91% | -4.83% | -5.75% |
| 4-AAP:1.5 | 1 | 210.3 | 204.7 | 201.3 | 200.1 | 197.5 | 195.6 |
| | 2 | 206.0 | 203.3 | 202.1 | 199.5 | 198.3 | 196.1 |
| | X | 208.2 | 204.0 | 201.7 | 199.8 | 197.9 | 195.9 |
| | RE/% | | -1.99% | -3.10% | -4.01% | -4.92% | -5.91% |
| 4-AAP:2.5 | 1 | 203.1 | 198.6 | 195.5 | 193.5 | 191.5 | 189.5 |
| | 2 | 201.2 | 199.5 | 196.1 | 194.2 | 192.1 | 190.2 |
| | X | 202.2 | 199.1 | 195.8 | 193.9 | 191.8 | 189.9 |
| | RE/% | | -1.53% | -3.14% | -4.11% | -5.12% | -6.08% |
| 4-AAP:5 | 1 | 204.6 | 200.3 | 198.5 | 195.2 | 192.4 | 189.4 |
| | 2 | 206.3 | 199.6 | 197.5 | 194.6 | 191.6 | 188.7 |
| | X | 205.5 | 200.0 | 198.0 | 194.9 | 192.0 | 189.1 |
| | RE/% | | -2.68% | -3.63% | -5.14% | -6.55% | -7.98% |
| | | | | | | | |
| DAOS | Calcium Dobesilate | 0 mg/L | 20 mg/L | 40 mg/L | 60 mg/L | 80 mg/L | 100 mg/L |
| 4-AAP:0.25 | 1 | 207.6 | 202.3 | 200.1 | 196.7 | 194.3 | 192.1 |
| | 2 | 209.1 | 203.5 | 199.5 | 197.1 | 195.6 | 191.3 |
| | X | 208.4 | 202.9 | 199.8 | 196.9 | 195.0 | 191.7 |
| | RE/% | | -2.62% | -4.10% | -5.50% | -6.43% | -7.99% |
| 4-AAP:0.5 | 1 | 205.5 | 203.1 | 200.4 | 199.3 | 198.6 | 196.5 |
| | 2 | 204.3 | 202.5 | 201.3 | 199.5 | 197.8 | 197.1 |
| | X | 204.9 | 202.8 | 200.9 | 199.4 | 198.2 | 196.8 |
| | RE/% | | -1.02% | -1.98% | -2.68% | -3.27% | -3.95% |
| 4-AAP:1.0 | 1 | 206.2 | 202.1 | 198.5 | 197.6 | 195.3 | 193.6 |
| | 2 | 205.7 | 201.2 | 199.1 | 196.9 | 194.9 | 193.2 |
| | X | 206.0 | 201.7 | 198.8 | 197.3 | 195.1 | 193.4 |
| | RE/% | | -2.09% | -3.47% | -4.22% | -5.27% | -6.09% |
| 4-AAP:1.5 | 1 | 208.3 | 203.1 | 200.1 | 197.6 | 196.1 | 195.1 |
| | 2 | 206.1 | 202.5 | 199.5 | 198.3 | 196.8 | 194.8 |
| | X | 207.2 | 202.8 | 199.8 | 198.0 | 196.5 | 195.0 |
| | RE/% | | -2.12% | -3.57% | -4.46% | -5.19% | -5.91% |
| 4-AAP:2.5 | 1 | 203.1 | 197.8 | 195.6 | 193.6 | 191.8 | 190.1 |
| | 2 | 201.2 | 196.9 | 194.8 | 192.9 | 191.2 | 189.8 |
| | X | 202.2 | 197.4 | 195.2 | 193.3 | 191.5 | 190.0 |
| | RE/% | | -2.37% | -3.44% | -4.40% | -5.27% | -6.04% |

(continued)

| DAOS | Calcium Dobesilate | 0 mg/L | 20 mg/L | 40 mg/L | 60 mg/L | 80 mg/L | 100 mg/L |
|---|---|---|---|---|---|---|---|
| 4-AAP:5 | 1 | 202.8 | 195.5 | 193.1 | 190.5 | 189.5 | 186.1 |
| | 2 | 202.1 | 196.6 | 192.5 | 191.2 | 188.7 | 187.1 |
| | X | 202.5 | 196.1 | 192.8 | 190.9 | 189.1 | 186.6 |
| | RE/% | | -3.16% | -4.77% | -5.73% | -6.59% | -7.83% |

| HDAOS | Calcium Dobesilate | 0 mg/L | 20 mg/L | 40 mg/L | 60 mg/L | 80 mg/L | 100 mg/L |
|---|---|---|---|---|---|---|---|
| 0.1mmol/L | 1 | 240.6 | 229.3 | 227.5 | 225.1 | 222.1 | 218.5 |
| | 2 | 235.4 | 230.1 | 226.8 | 224.3 | 221.5 | 219.6 |
| | X | 238.0 | 229.7 | 227.2 | 224.7 | 221.8 | 219.1 |
| | RE/% | | -3.49% | -4.56% | -5.59% | -6.81% | -7.96% |
| 0.3mmol/L | 1 | 241.8 | 234.5 | 231.2 | 228.6 | 226.5 | 224.3 |
| | 2 | 236.9 | 233.9 | 230.5 | 227.9 | 226.9 | 225.4 |
| | X | 239.4 | 234.2 | 230.9 | 228.3 | 226.7 | 224.9 |
| | RE/% | | -2.15% | -3.55% | -4.64% | -5.29% | -6.06% |
| 10mmol/L | 1 | 238.8 | 235.6 | 232.1 | 230.5 | 228.9 | 228.1 |
| | 2 | 236.9 | 234.3 | 231.5 | 231.1 | 229.5 | 228.5 |
| | X | 237.9 | 235.0 | 231.8 | 230.8 | 229.2 | 228.3 |
| | RE/% | | -1.22% | -2.54% | -2.96% | -3.64% | -4.02% |
| 20mmol/L | 1 | 236.3 | 228.1 | 226.6 | 225.3 | 222.3 | 219.8 |
| | 2 | 231.6 | 229.5 | 227.1 | 224.9 | 221.6 | 220.1 |
| | X | 234.0 | 228.8 | 226.9 | 225.1 | 222.0 | 220.0 |
| | RE/% | | -2.20% | -3.03% | -3.78% | -5.13% | -5.98% |
| 50mmol/L | 1 | 239.3 | 231.3 | 228.4 | 227.6 | 225.4 | 223.3 |
| | 2 | 234.6 | 232.5 | 229.5 | 226.1 | 224.9 | 223.2 |
| | X | 237.0 | 231.9 | 229.0 | 226.9 | 225.2 | 223.3 |
| | RE/% | | -2.13% | -3.38% | -4.26% | -4.98% | -5.78% |
| 60mmol/L | 1 | 240.3 | 232.4 | 229.4 | 227.1 | 223.1 | 221.1 |
| | 2 | 238.5 | 231.5 | 228.9 | 226.5 | 224.3 | 219.2 |
| | X | 239.4 | 232.0 | 229.2 | 226.8 | 223.7 | 220.2 |
| | RE/% | | -3.11% | -4.28% | -5.26% | -6.56% | -8.04% |

| DAOS | Calcium Dobesilate | 0 mg/L | 20 mg/L | 40 mg/L | 60 mg/L | 80 mg/L | 100 mg/L |
|---|---|---|---|---|---|---|---|
| 0.1mmol/L | 1 | 235.1 | 226.4 | 223.3 | 221.3 | 218.6 | 214.8 |
| | 2 | 230.2 | 225.1 | 224.1 | 220.1 | 217.6 | 213.6 |
| | X | 232.7 | 225.8 | 223.7 | 220.7 | 218.1 | 214.2 |
| | RE/% | | -2.97% | -3.85% | -5.14% | -6.25% | -7.93% |

(continued)

| DAOS | Calcium Dobesilate | 0 mg/L | 20 mg/L | 40 mg/L | 60 mg/L | 80 mg/L | 100 mg/L |
|---|---|---|---|---|---|---|---|
| 0.3mmol/L | 1 | 236.3 | 229.1 | 227.8 | 226.3 | 224.3 | 222.1 |
| | 2 | 235.2 | 230.5 | 228.2 | 226.8 | 223.6 | 221.6 |
| | X | 235.8 | 229.8 | 228.0 | 226.6 | 224.0 | 221.9 |
| | RE/% | | -2.52% | -3.29% | -3.90% | -5.01% | -5.90% |
| 10mmol/L | 1 | 234.3 | 227.5 | 226.4 | 225.3 | 223.6 | 223.1 |
| | 2 | 229.6 | 228.5 | 226.9 | 224.8 | 224.1 | 222.5 |
| | X | 232.0 | 228.0 | 226.7 | 225.1 | 223.9 | 222.8 |
| | RE/% | | -1.70% | -2.28% | -2.97% | -3.49% | -3.94% |
| 20mmol/L | 1 | 237.3 | 228.3 | 227.6 | 225.4 | 223.6 | 221.1 |
| | 2 | 232.6 | 229.6 | 226.9 | 224.8 | 222.4 | 220.3 |
| | X | 235.0 | 229.0 | 227.3 | 225.1 | 223.0 | 220.7 |
| | RE/% | | -2.55% | -3.28% | -4.19% | -5.09% | -6.07% |
| 50mmol/L | 1 | 235.8 | 226.7 | 225.4 | 223.2 | 221.4 | 219.6 |
| | 2 | 231.3 | 227.6 | 224.2 | 222.8 | 220.9 | 218.5 |
| | X | 233.6 | 227.2 | 224.8 | 223.0 | 221.2 | 219.1 |
| | RE/% | | -2.74% | -3.75% | -4.52% | -5.31% | -6.21% |
| 60mmol/L | 1 | 234.1 | 224.5 | 221.6 | 220.1 | 217.5 | 215.3 |
| | 2 | 232.5 | 223.9 | 222.1 | 219.6 | 218.1 | 214.1 |
| | X | 233.3 | 224.2 | 221.9 | 219.9 | 217.8 | 214.7 |
| | RE/% | | -3.90% | -4.91% | -5.77% | -6.64% | -7.97% |
| | | | | | | | |
| HDAOS | Calcium Dobesilate | 0 mg/L | 20 mg/L | 40 mg/L | 60 mg/L | 80 mg/L | 100 mg/L |
| R2:creatininase 105KU/L | 1 | 190.3 | 187.1 | 183.3 | 182.6 | 180.3 | 179.6 |
| | 2 | 191.2 | 186.3 | 184.9 | 181.9 | 181.1 | 180.1 |
| | X | 190.8 | 186.7 | 184.1 | 182.3 | 180.7 | 179.9 |
| | RE/% | | -2.12% | -3.49% | -4.46% | -5.27% | -5.71% |
| R2:creatininase 120KU/L | 1 | 192.4 | 190.3 | 188.1 | 186.2 | 185.1 | 183.9 |
| | 2 | 191.6 | 189.4 | 187.6 | 186.9 | 186.2 | 184.3 |
| | X | 192.0 | 189.9 | 187.9 | 186.6 | 185.7 | 184.1 |
| | RE/% | | -1.12% | -2.16% | -2.84% | -3.31% | -4.11% |
| R2:creatininase300KU/L | 1 | 189.4 | 186.7 | 185.3 | 184.3 | 183.6 | 181.9 |
| | 2 | 189.6 | 187.1 | 186.1 | 183.6 | 182.3 | 182.0 |
| | X | 189.5 | 186.9 | 185.7 | 184.0 | 183.0 | 182.0 |
| | RE/% | | -1.37% | -2.01% | -2.93% | -3.46% | -3.98% |
| R2:creatininase500KU/L | 1 | 192.6 | 190.1 | 188.1 | 186.8 | 185.9 | 184.9 |
| | 2 | 193.2 | 189.5 | 187.9 | 187.1 | 186.2 | 185.3 |
| | X | 192.9 | 189.8 | 188.0 | 187.0 | 186.1 | 185.1 |
| | RE/% | | -1.61% | -2.54% | -3.08% | -3.55% | -4.04% |

(continued)

| HDAOS | Calcium Dobesilate | 0 mg/L | 20 mg/L | 40 mg/L | 60 mg/L | 80 mg/L | 100 mg/L |
|---|---|---|---|---|---|---|---|
| R2:creatininase800KU/L | 1 | 191.4 | 188.8 | 186.3 | 185.4 | 183.6 | 181.3 |
| | 2 | 192.2 | 189.3 | 186.1 | 184.9 | 183.1 | 182.0 |
| | X | 191.8 | 189.1 | 186.2 | 185.2 | 183.4 | 181.7 |
| | RE/% | | -1.43% | -2.92% | -3.47% | -4.41% | -5.29% |

| DAOS | Calcium Dobesilate | 0 mg/L | 20 mg/L | 40 mg/L | 60 mg/L | 80 mg/L | 100 mg/L |
|---|---|---|---|---|---|---|---|
| R2:creatininase 105KU/L | 1 | 175.2 | 171.4 | 169.2 | 167.6 | 166.5 | 164.8 |
| | 2 | 174.9 | 170.6 | 168.5 | 168.1 | 165.9 | 164.5 |
| | X | 175.1 | 171.0 | 168.9 | 167.9 | 166.2 | 164.7 |
| | RE/% | | -2.31 % | -3.54% | -4.11% | -5.06% | -5.94% |
| R2:creatininase 120KU/L | 1 | 175.6 | 174.3 | 172.5 | 171.1 | 169.9 | 168.9 |
| | 2 | 176.2 | 173.6 | 172.8 | 170.9 | 170.2 | 169.1 |
| | X | 175.9 | 174.0 | 172.7 | 171.0 | 170.1 | 169.0 |
| | RE/% | | -1.11% | -1.85% | -2.79% | -3.33% | -3.92% |
| R2:creatininase 300KU/L | 1 | 175.4 | 171.5 | 170.6 | 169.5 | 168.2 | 167.1 |
| | 2 | 173.8 | 172.6 | 171.3 | 169.8 | 169.0 | 167.8 |
| | X | 174.6 | 172.1 | 171.0 | 169.7 | 168.6 | 167.5 |
| | RE/% | | -1.46% | -2.09% | -2.84% | -3.44% | -4.10% |
| R2:creatininase 500KU/L | 1 | 175.6 | 172.5 | 171.6 | 170.4 | 169.5 | 167.9 |
| | 2 | 174.9 | 173.1 | 172.0 | 170.6 | 168.9 | 168.5 |
| | X | 175.3 | 172.8 | 171.8 | 170.5 | 169.2 | 168.2 |
| | RE/% | | -1.40% | -1.97% | -2.71% | -3.45% | -4.02% |
| R2:creatininase 800KU/L | 1 | 170.5 | 168.2 | 166.2 | 163.4 | 161.4 | 160.5 |
| | 2 | 171.6 | 167.4 | 165.3 | 162.8 | 162.0 | 161.2 |
| | X | 171.1 | 167.8 | 165.8 | 163.1 | 161.7 | 160.9 |
| | RE/% | | -1.90% | -3.10% | -4.65% | -5.47% | -5.96% |

| HDAOS | Calcium Dobesilate | 0 mg/L | 20 mg/L | 40 mg/L | 60 mg/L | 80 mg/L | 100 mg/L |
|---|---|---|---|---|---|---|---|
| R1:CAT 20KU/L | 1 | 195.8 | 192.3 | 190.5 | 188.1 | 186.3 | 184.3 |
| | 2 | 196.3 | 191.6 | 189.6 | 187.6 | 185.4 | 184.1 |
| | X | 196.1 | 192.0 | 190.1 | 187.9 | 185.9 | 184.2 |
| | RE/% | | -2.09% | -3.06% | -4.18% | -5.20% | -6.04% |
| R1: CAT 50KU/L | 1 | 193.5 | 190.5 | 189.6 | 188.8 | 187.1 | 186.5 |
| | 2 | 194.2 | 191.2 | 189.4 | 187.9 | 186.8 | 185.4 |
| | X | 193.9 | 190.9 | 189.5 | 188.4 | 187.0 | 186.0 |
| | RE/% | | -1.55% | -2.24% | -2.84% | -3.56% | -4.08% |

(continued)

| HDAOS | Calcium Dobesilate | 0 mg/L | 20 mg/L | 40 mg/L | 60 mg/L | 80 mg/L | 100 mg/L |
|---|---|---|---|---|---|---|---|
| R1:CAT 400KU/L | 1 | 192.4 | 190.2 | 188.6 | 188.0 | 186.6 | 185.4 |
| | 2 | 193.6 | 189.6 | 188.9 | 187.6 | 186.7 | 185.2 |
| | X | 193.0 | 189.9 | 188.8 | 187.8 | 186.7 | 185.3 |
| | RE/% | | -1.61% | -2.20% | -2.69% | -3.29% | -3.99% |
| R1:CAT 600KU/L | 1 | 195.4 | 192.4 | 191.4 | 190.1 | 189.1 | 187.6 |
| | 2 | 195.6 | 193.1 | 191.2 | 189.6 | 188.9 | 187.5 |
| | X | 195.5 | 192.8 | 191.3 | 189.9 | 189.0 | 187.6 |
| | RE/% | | -1.41% | -2.15% | -2.89% | -3.32% | -4.07% |
| R1:CAT 1000KU/L | 1 | 193.4 | 188.5 | 186.5 | 185.4 | 183.2 | 181.7 |
| | 2 | 194.2 | 189.3 | 187.3 | 184.3 | 182.8 | 182.1 |
| | X | 193.8 | 188.9 | 186.9 | 184.9 | 183.0 | 181.9 |
| | RE/% | | -2.53% | -3.56% | -4.62% | -5.57% | -6.14% |
| | | | | | | | |
| DAOS | Calcium Dobesilate | 0 mg/L | 20 mg/L | 40 mg/L | 60 mg/L | 80 mg/L | 100 mg/L |
| R1:CAT 10KU/L | 1 | 223.6 | 215.6 | 213.7 | 211.6 | 209.6 | 206.4 |
| | 2 | 224.7 | 216.3 | 214.5 | 210.7 | 208.7 | 205.3 |
| | X | 224.2 | 216.0 | 214.1 | 211.2 | 209.2 | 205.9 |
| | RE/% | | -3.66% | -4.48% | -5.80% | -6.69% | -8.16% |
| R1:CAT 20KU/L | 1 | 193.4 | 190.5 | 188.5 | 185.3 | 183.6 | 182.9 |
| | 2 | 195.3 | 189.6 | 187.6 | 186.1 | 184.1 | 183.1 |
| | X | 194.4 | 190.1 | 188.1 | 185.7 | 183.9 | 183.0 |
| | RE/% | | -2.21% | -3.24% | -4.45% | -5.40% | -5.84% |
| R1:CAT 50KU/L | 1 | 193.4 | 191.3 | 188.9 | 187.5 | 186.5 | 185.1 |
| | 2 | 193.3 | 190.6 | 189.6 | 187.9 | 186.8 | 186.2 |
| | X | 193.4 | 191.0 | 189.3 | 187.7 | 186.7 | 185.7 |
| | RE/% | | -1.24% | -2.12% | -2.92% | -3.47% | -3.98% |
| R1:CAT 400KU/L | 1 | 194.4 | 191.3 | 190.5 | 189.6 | 188.6 | 186.9 |
| | 2 | 195.1 | 192.4 | 190.8 | 190.1 | 187.9 | 187.0 |
| | X | 194.8 | 191.9 | 190.7 | 189.9 | 188.3 | 187.0 |
| | RE/% | | -1.49% | -2.11% | -2.52% | -3.34% | -4.01% |
| R1:CAT 600KU/L | 1 | 195.1 | 192.1 | 190.4 | 189.4 | 187.9 | 187.4 |
| | 2 | 194.8 | 191.6 | 190.6 | 188.9 | 188.1 | 186.9 |
| | X | 195.0 | 191.9 | 190.5 | 189.2 | 188.0 | 187.2 |
| | RE/% | | -1.59% | -2.28% | -2.98% | -3.57% | -4.00% |
| R1: CAT 1000KU/L | 1 | 190.4 | 185.6 | 184.3 | 182.9 | 180.4 | 179.6 |
| | 2 | 191.2 | 186.7 | 183.6 | 181.5 | 180.6 | 178.6 |
| | X | 190.8 | 186.15 | 183.95 | 182.2 | 180.5 | 179.1 |
| | RE/% | | -2.44% | -3.59% | -4.51% | -5.40% | -6.13% |

(continued)

| HDAOS | Calcium Dobesilate | 0 mg/L | 20 mg/L | 40 mg/L | 60 mg/L | 80 mg/L | 100 mg/L |
|---|---|---|---|---|---|---|---|
| sarcosine oxidase: | 1 | 240.2 | 237.6 | 235.2 | 233.8 | 232.1 | 230.4 |
| | 0.5KU/L 2 | 239.5 | 236.7 | 234.5 | 233.2 | 231.9 | 230.5 |
| | X | 239.9 | 237.2 | 234.9 | 233.5 | 232.0 | 230.5 |
| | RE/% | / | -1.13% | -2.08% | -2.65% | -3.27% | -3.92% |
| sarcosine oxidase:2KU/L | 1 | 238.5 | 234.1 | 232.8 | 231.6 | 230.5 | 229.1 |
| | 2 | 237.6 | 235.9 | 233.9 | 232.1 | 230.8 | 228.1 |
| | X | 238.1 | 235.0 | 233.4 | 231.9 | 230.7 | 228.6 |
| | RE/% | / | -1.28% | -1.97% | -2.60% | -3.11% | -3.97% |
| sarcosine oxidase:50KU/L | 1 | 241.3 | 237.6 | 235.7 | 234.1 | 232.5 | 230.6 |
| | 2 | 240.5 | 238.4 | 236.4 | 233.9 | 231.8 | 230.9 |
| | X | 240.9 | 238.0 | 236.1 | 234.0 | 232.2 | 230.8 |
| | RE/% | / | -1.20% | -2.01 % | -2.86% | -3.63% | -4.21% |
| sarcosine oxidase: 100KU/L | 1 | 235.2 | 233.5 | 231.6 | 229.4 | 227.3 | 226.5 |
| | 2 | 236.4 | 232.6 | 230.9 | 228.7 | 227.9 | 225.8 |
| | X | 235.8 | 233.1 | 231.3 | 229.1 | 227.6 | 226.2 |
| | RE/% | / | -1.17% | -1.93% | -2.86% | -3.48% | -4.09% |
| sarcosine oxidase:150KU/L | 1 | 238.4 | 235.9 | 234.2 | 232.4 | 230.4 | 229.5 |
| | 2 | 238.1 | 236.1 | 233.6 | 232.9 | 231.1 | 228.7 |
| | X | 238.3 | 236.0 | 233.9 | 232.7 | 230.8 | 229.1 |
| | RE/% | / | -0.94% | -1.83% | -2.35% | -3.15% | -3.84% |

| DAOS | Calcium Dobesilate | 0 mg/L | 20 mg/L | 40 mg/L | 60 mg/L | 80 mg/L | 100 mg/L |
|---|---|---|---|---|---|---|---|
| sarcosine oxidase:0.5KU/L | 1 | 235.2 | 233.5 | 231.5 | 229.4 | 227.1 | 226.5 |
| | 2 | 236.5 | 232.1 | 230.4 | 228.7 | 227.8 | 226.1 |
| | X | 235.9 | 232.8 | 231.0 | 229.1 | 227.5 | 226.3 |
| | RE/% | / | -1.29% | -2.08% | -2.88% | -3.56% | -4.05% |
| sarcosine oxidase:2KU/L | 1 | 230.4 | 227.4 | 226.3 | 224.3 | 223.8 | 222.1 |
| | 2 | 232.1 | 228.5 | 225.4 | 224.8 | 222.9 | 221.8 |
| | X | 231.3 | 228.0 | 225.9 | 224.6 | 223.4 | 222.0 |
| | RE/% | / | -1.43% | -2.34% | -2.90% | -3.42% | -4.02% |
| sarcosine oxidase:50KU/L | 1 | 231.3 | 228.1 | 226.3 | 224.6 | 223.1 | 221.5 |
| | 2 | 229.5 | 227.5 | 225.9 | 224.1 | 222.5 | 220.9 |
| | X | 230.4 | 227.8 | 226.1 | 224.4 | 222.8 | 221.2 |
| | RE/% | / | -1.13% | -1.87% | -2.63% | -3.30% | -3.99% |
| sarcosine oxidase: 100KU/L | 1 | 233.2 | 230.0 | 228.4 | 226.5 | 225.3 | 223.7 |
| | 2 | 232.4 | 229.4 | 227.6 | 226.8 | 224.8 | 222.9 |
| | X | 232.8 | 229.7 | 228.0 | 226.7 | 225.1 | 223.3 |
| | RE/% | / | -1.33% | -2.06% | -2.64% | -3.33% | -4.08% |

(continued)

| DAOS | Calcium Dobesilate | 0 mg/L | 20 mg/L | 40 mg/L | 60 mg/L | 80 mg/L | 100 mg/L |
|---|---|---|---|---|---|---|---|
| sarcosine oxidase:150KU/L | 1 | 234.4 | 231.5 | 230.6 | 228.4 | 226.5 | 225.6 |
| | 2 | 235.1 | 232.5 | 229.7 | 227.6 | 227.1 | 225.1 |
| | X | 234.8 | 232.0 | 230.2 | 228.0 | 226.8 | 225.4 |
| | RE/% | / | -1.17% | -1.96% | -2.88% | -3.39% | -4.00% |

| HDAOS | Calcium Dobesilate | 0 mg/L | 20 mg/L | 40 mg/L | 60 mg/L | 80 mg/L | 100 mg/L |
|---|---|---|---|---|---|---|---|
| creatinase: 1KU/L | 1 | 235.6 | 233.4 | 231.5 | 229.6 | 227.6 | 226.5 |
| | 2 | 236.1 | 232.8 | 230.6 | 228.4 | 228.1 | 225.9 |
| | X | 235.9 | 233.1 | 231.1 | 229.0 | 227.9 | 226.2 |
| | RE/% | / | -1.17% | -2.04% | -2.90% | -3.39% | -4.09% |
| creatinase: 5KU/L | 1 | 234.5 | 231.5 | 230.4 | 229.5 | 227.3 | 225.9 |
| | 2 | 235.1 | 232.4 | 230.2 | 228.4 | 226.5 | 224.9 |
| | X | 234.8 | 232.0 | 230.3 | 229.0 | 226.9 | 225.4 |
| | RE/% | / | -1.21% | -1.92% | -2.49% | -3.36% | -4.00% |
| creatinase: 75KU/L | 1 | 237.3 | 234.3 | 232.8 | 230.4 | 228.6 | 227.3 |
| | 2 | 236.6 | 234.1 | 231.6 | 229.5 | 229.1 | 228.1 |
| | X | 237.0 | 234.2 | 232.2 | 230.0 | 228.9 | 227.7 |
| | RE/% | / | -1.16% | -2.00% | -2.95% | -3.42% | -3.90% |
| creatinase: 150KU/L | 1 | 235.8 | 233.1 | 230.5 | 229.6 | 227.6 | 225.9 |
| | 2 | 235.4 | 232.6 | 229.9 | 228.7 | 226.8 | 226.1 |
| | X | 235.6 | 232.9 | 230.2 | 229.2 | 227.2 | 226.0 |
| | RE/% | / | -1.17% | -2.29% | -2.74% | -3.57% | -4.07% |
| creatinase: 300KU/L | 1 | 234.2 | 231.6 | 228.9 | 227.5 | 226.5 | 224.6 |
| | 2 | 234.6 | 232.0 | 229.1 | 228.1 | 225.6 | 225.4 |
| | X | 234.4 | 231.8 | 229.0 | 227.8 | 226.1 | 225.0 |
| | RE/% | / | -1.11% | -2.30% | -2.82% | -3.56% | -4.01% |

| DAOS | Calcium Dobesilate | 0 mg/L | 20 mg/L | 40 mg/L | 60 mg/L | 80 mg/L | 100 mg/L |
|---|---|---|---|---|---|---|---|
| creatinase: 1KU/L | 1 | 234.2 | 231.6 | 228.7 | 228.4 | 226.3 | 224.9 |
| | 2 | 233.8 | 230.9 | 229.5 | 227.1 | 227.1 | 224.5 |
| | X | 234.0 | 231.3 | 229.1 | 227.8 | 226.7 | 224.7 |
| | RE/% | / | -1.18% | -2.09% | -2.67% | -3.12% | -3.97% |
| creatinase: 5KU/L | 1 | 232.3 | 229.4 | 227.6 | 225.4 | 224.8 | 223.2 |
| | 2 | 233.1 | 230.8 | 226.8 | 226.1 | 223.9 | 223.9 |
| | X | 232.7 | 230.1 | 227.2 | 225.8 | 224.4 | 223.6 |
| | RE/% | / | -1.12% | -2.36% | -2.99% | -3.59% | -3.93% |

(continued)

| DAOS | Calcium Dobesilate | 0 mg/L | 20 mg/L | 40 mg/L | 60 mg/L | 80 mg/L | 100 mg/L |
|---|---|---|---|---|---|---|---|
| creatinase: 75KU/L | 1 | 231.4 | 228.4 | 226.5 | 224.3 | 223.5 | 221.9 |
| | 2 | 230.6 | 227.6 | 225.9 | 223.8 | 223.2 | 221.4 |
| | X | 231.0 | 228.0 | 226.2 | 224.1 | 223.4 | 221.7 |
| | RE/% | / | -1.30% | -2.08% | -3.01% | -3.31 % | -4.05% |
| creatinase: 150KU/L | 1 | 232.5 | 230.5 | 227.6 | 226.9 | 225.1 | 223.6 |
| | 2 | 232.7 | 229.6 | 228.1 | 226.2 | 226.1 | 223.1 |
| | X | 232.6 | 230.1 | 227.9 | 226.6 | 225.6 | 223.4 |
| | RE/% | / | -1.10% | -2.04% | -2.60% | -3.01% | -3.98% |
| creatinase: 300KU/L | 1 | 233.4 | 230.1 | 227.6 | 226.5 | 224.3 | 223.6 |
| | 2 | 232.1 | 229.5 | 228.1 | 225.9 | 225.1 | 223.2 |
| | X | 232.8 | 229.8 | 227.9 | 226.2 | 224.7 | 223.4 |
| | RE/% | / | -1.27% | -2.11% | -2.81 % | -3.46% | -4.02% |

| HDAOS | Calcium Dobesilate | 0 mg/L | 20 mg/L | 40 mg/L | 60 mg/L | 80 mg/L | 100 mg/L |
|---|---|---|---|---|---|---|---|
| peroxidase: 1KU/L | 1 | 220.6 | 218.6 | 215.3 | 214.3 | 213.2 | 211.9 |
| | 2 | 221.1 | 216.7 | 215.9 | 213.9 | 212.6 | 212.1 |
| | X | 220.9 | 217.7 | 215.6 | 214.1 | 212.9 | 212.0 |
| | RE/% | / | -1.45% | -2.38% | -3.06% | -3.60% | -4.01% |
| peroxidase: 25KU/L | 1 | 221.5 | 219.6 | 217.1 | 216.1 | 214.6 | 212.7 |
| | 2 | 221.3 | 218.2 | 216.9 | 215.4 | 213.9 | 212.6 |
| | X | 221.4 | 218.9 | 217.0 | 215.8 | 214.3 | 212.7 |
| | RE/% | / | -1.13% | -1.99% | -2.55% | -3.23% | -3.95% |
| peroxidase: 50KU/L | 1 | 220.3 | 218.1 | 216.2 | 213.6 | 212.4 | 211.6 |
| | 2 | 220.1 | 217.3 | 215.4 | 214.1 | 213.0 | 211.1 |
| | X | 220.2 | 217.7 | 215.8 | 213.9 | 212.7 | 211.4 |
| | RE/% | / | -1.14% | -2.00% | -2.88% | -3.41% | -4.02% |
| peroxidase: 100KU/L | 1 | 219.5 | 216.7 | 214.3 | 212.9 | 212.1 | 210.9 |
| | 2 | 218.8 | 215.6 | 213.2 | 212.4 | 211.6 | 209.9 |
| | X | 219.2 | 216.2 | 213.8 | 212.7 | 211.9 | 210.4 |
| | RE/% | / | -1.37% | -2.46% | -2.97% | -3.33% | -3.99% |

| DAOS | Calcium Dobesilate | 0 mg/L | 20 mg/L | 40 mg/L | 60 mg/L | 80 mg/L | 100 mg/L |
|---|---|---|---|---|---|---|---|
| peroxidase: 1KU/L | 1 | 218.1 | 214.5 | 213.4 | 211.6 | 211.0 | 209.6 |
| | 2 | 217.8 | 215.3 | 212.9 | 212.4 | 210.9 | 208.7 |
| | X | 218.0 | 214.9 | 213.2 | 212.0 | 211.0 | 209.2 |
| | RE/% | / | -1.40% | -2.20% | -2.73% | -3.21% | -4.04% |

(continued)

| DAOS | Calcium Dobesilate | 0 mg/L | 20 mg/L | 40 mg/L | 60 mg/L | 80 mg/L | 100 mg/L |
|---|---|---|---|---|---|---|---|
| peroxidase: 25KU/L | 1 | 217.4 | 214.3 | 212.3 | 210.6 | 209.6 | 208.6 |
| | 2 | 216.9 | 213.6 | 211.9 | 211.3 | 210.1 | 208.3 |
| | X | 217.2 | 214.0 | 212.1 | 211.0 | 209.9 | 208.5 |
| | RE/% | / | -1.47% | -2.33% | -2.86% | -3.36% | -4.01% |
| peroxidase: 50KU/L | 1 | 218.1 | 214.3 | 213.4 | 212.5 | 210.3 | 209.6 |
| | 2 | 218.6 | 215.4 | 212.9 | 211.4 | 211.4 | 210.1 |
| | X | 218.4 | 214.9 | 213.2 | 212.0 | 210.9 | 209.9 |
| | RE/% | / | -1.60% | -2.38% | -2.93% | -3.43% | -3.89% |
| peroxidase: 100KU/L | 1 | 217.5 | 215.6 | 212.6 | 210.6 | 210.1 | 209.4 |
| | 2 | 218.1 | 214.1 | 213.1 | 211.8 | 210.5 | 208.9 |
| | X | 217.8 | 214.9 | 212.9 | 211.2 | 210.3 | 209.2 |
| | RE/% | / | -1.35% | -2.27% | -3.03% | -3.44% | -3.97% |

**2. Comparison with commercially available kits**

**2.1** Comparison with commercially available kit 1

Components of the commercially available kit 1 are described in Section 6.1.1 of Example 6. The detection was carried out according to the method described above. The results are shown in Table 17 below:

**Table 17:** Effects of commercially available kit 1 on the interference of anti-Calcium Dobesilate interference

| Calcium Dobesilate concentration | 0 mg/L | 20 mg/L | 40 mg/L | 60 mg/L | 80 mg/L | 100 mg/L |
|---|---|---|---|---|---|---|
| 1 | 143.3 | 109.8 | 88.8 | 71.9 | 60.2 | 50.6 |
| 2 | 143.3 | 110.3 | 89.2 | 72.2 | 63.6 | 57.9 |
| M | 143.3 | 110.1 | 89.0 | 72.1 | 61.9 | 54.3 |
| RE/% | / | -23.20% | -37.89% | -49.72% | -56.80% | -62.14% |
| Interference or not | / | Yes | Yes | Yes | Yes | Yes |

It can be seen that the commercially available kit 1 can not resist the interference of calcium dobesilate in the detection of creatinine.

**2.2** Comparison with commercially available kit 2

Components of the commercially available kit 2 are described in Section 5.2.1 of Example 6. The detection was carried out according to the method described above. The results are shown in Table 18 below:

**Table 18:** Effects of commercially available kit 2 on the interference of anti-Calcium Dobesilate interference

| Calcium Dobesilate concentration | 0 mg/L | 20 mg/L | 40 mg/L | 60 mg/L | 80 mg/L | 100 mg/L |
|---|---|---|---|---|---|---|
| 1 | 177.5 | 144.1 | 123.5 | 106.4 | 95.8 | 88.1 |
| 2 | 175.8 | 144.7 | 121.6 | 106.4 | 96.6 | 88.8 |
| M | 176.7 | 144.4 | 122.6 | 106.4 | 96.2 | 88.5 |
| RE/% | / | -18.26% | -30.63% | -39.77% | -45.54% | -49.93% |
| Interference or not | / | Yes | Yes | Yes | Yes | Yes |

It can be seen that the commercially available kit 2 can not resist the interference of calcium dobesilate in the detection of creatinine.

**Claims**

1.  An *in vitro* use of a kit for quantifying creatinine amount in a biological sample which contains calcium dobesilate and/ or etamsylate, wherein said kit comprises a first reagent set and a second reagent set,
    said first reagent set containing the chromogen selected from N- (2-hydroxy-3-sulfopropyl) -3,5-dimethoxyaniline or its salts, N-ethyl-N- (2-hydroxy-3-sulfopropyl) - 3,5-dimethoxyaniline or its salts, and combination thereof, and said second reagent set containing 4-aminoantipyrine.

2.  The *in vitro* use of the kit according to claim 1, wherein said chromogen is N-ethyl-N-(2-hydroxy-3-sulfopropyl)-3,5-dimethoxyaniline sodium salt or N-(2-Hydroxy-3-sulfopropyl)-3,5-dimethoxyaniline sodium salt.

3.  The *in vitro* use of the kit according to any one of the preceding claims, wherein said first reagent set further contains creatinase, sarcosine oxidase and catalase, and said second reagent set further contains creatininase and peroxidase.

4.  The *in vitro* use of the kit according to claim 1 or 2, wherein the concentration of 4-aminoantipyrine is 0.25-5 g / L, preferably 0.5-2.5 g/L.

5.  The *in vitro* use of the kit according to any one of the preceding claims, wherein the concentration of creatininase is 105-800 KU/L, preferably 120-500 KU/L.

6.  The *in vitro* use of the kit according to any one of the preceding claims, wherein the concentration of catalase is 20-1000 KU/L, preferably 50-600 KU/L.

7.  The *in vitro* use of the kit according to any one of the preceding claims, wherein the concentration of chromogen is 0.1-60 mmol/L, preferably 0.3-50 mmol/L.

8.  The *in vitro* use of the kit according to any one of the preceding claims, wherein the concentration of creatinase is 1-300 KU / L, preferably 5-150 KU / L; the concentration of sarcosine oxidase is 0.5-150 KU/L, preferably 2-100 KU/L; and the concentration of the peroxidase is 1-100 KU / L, preferably 1-50 KU / L.

9.  The *in vitro* use of the kit according to any one of the preceding claims, wherein said first reagent set and /or said second reagent set further comprises a buffer solution selected from the group consisting of MOPS Buffer, good's Buffer, TRIS Buffer, phosphate Buffer and glycine Buffer, and any combination thereof.

10. The *in vitro* use of the kit according to any one of the preceding claims, wherein said first reagent set further comprises one or more of the following agents: a stabilizer, a preservative, a surfactant and an ascorbic acid oxidase.

11. The *in vitro* use of the kit according to any one of the preceding claims, wherein said second reagent set further comprises a preservative and/or a surfactant.

12. The *in vitro* use of the kit according to any one of the preceding claims, wherein said biological sample was previously obtained from a patient administered or being administered to calcium dobesilate and / or etamsylate.

13. The *in vitro* use of the kit according to any one of the preceding claims, wherein the creatinine amount in said biological sample is less than 10%, preferably less than 8%, compared to the creatinine amount measured against a control sample, more preferably less than 6% and most preferably less than 4%, the control sample being the same as said biological sample but not containing calcium dobesilate and etamsylate.

14. A method for *in vitro* quantifying the creatinine amount in a biological sample which contains dobesilate and/or etamsylate said method comprising :

    (a) a first step of:

(a1) on one hand, mixing a first reagent set with said biological sample, and measuring the absorbance $A1_U$ of the mixture, and
(a2) on the other hand, mixing a first reagent set with a calibrating agent, and measuring the absorbance $A1_C$ of the mixture,

(b) a second step of :

(b1) on one hand, mixing a second reagent set with the mixture obtained in step (a1) and then measuring the absorbance A2u of said mixture,
(b2) on the other hand, mixing a second reagent set with the mixture obtained in step (a2) and then measuring the absorbance $A2_C$ of said mixture,

(c) a step of calculating the creatinine amount according to the formula:

$$\text{creatinine concentration in the said biological sample} = (A2_U - A1_U) / (A2_C - A1_C) * C_C$$

wherein $C_C$ represents the concentration of the said calibrating agent,
the first reagent set containing a chromogen selected from N- (2-hydroxy-3-sulfopropyl) -3,5-dimethoxyaniline or its salts, N-ethyl-N- (2-hydroxy-3-sulfopropyl) -3,5-dimethoxyaniline or its salts, and combination thereof, and
the second reagent set containing 4-aminoantipyrine.

## Patentansprüche

1.  *In-vitro*-Verwendung eines Kits zum Quantifizieren einer Kreatininmenge in einer biologischen Probe, die Calcium-Dobesilat und/oder Etamsylat enthält, wobei das Kit einen ersten Reagenssatz und einen zweiten Reagenssatz umfasst,
    wobei der erste Reagenssatz das Chromogen enthält, das aus N-(2-hydroxy-3-sulfopropyl)-3,5-dimethoxyanilin oder seinen Salzen, N-Ethyl-N-(2-hydroxy-3-sulfopropyl)-3,5-dimethoxyanilin oder seinen Salzen, und einer Kombination davon ausgewählt ist, und der zweite Reagenssatz 4-Aminoantipyrin enthält.

2.  *In-vitro*-Verwendung des Kits nach Anspruch 1, wobei das Chromogen N-Ethyl-N-(2-hydroxy-3-sulfopropyl)-3,5-dimethoxyanilin-Natriumsalz oder N-(2-hydroxy- 3-sulfopropyl)-3,5-dimethoxyanilin-Natriumsalz ist.

3.  *In-vitro*-Verwendung des Kits nach einem der vorhergehenden Ansprüche, wobei der erste Reagenssatz ferner Kreatinase, Sarcosinoxidase und Katalase enthält und der zweite Reagenssatz ferner Kreatininase und Peroxidase enthält.

4.  *In-vitro*-Verwendung des Kits nach Anspruch 1 oder 2, wobei die Konzentration von 4-Aminoantipyrin 0,25-5 g/l, vorzugsweise 0,5-2,5 g/l, ist.

5.  *In-vitro*-Verwendung des Kits nach einem der vorhergehenden Ansprüche, wobei die Konzentration der Kreatininase 105-800 KU/l, vorzugsweise 120-500 KU/l, ist.

6.  *In-vitro*-Verwendung des Kits nach einem der vorhergehenden Ansprüche, wobei die Konzentration der Katalase 20-1000 KU/l, vorzugsweise 50-600 KU/l, ist.

7.  *In-vitro-Verwendung* des Kits nach einem der vorhergehenden Ansprüche, wobei die Konzentration des Chromogens 0,1-60 mmol/l, vorzugsweise 0,3-50 mmol/l, ist.

8.  *In-vitro*-Verwendung des Kits nach einem der vorhergehenden Ansprüche, wobei die Konzentration der Kreatinase 1-300 KU/l, vorzugsweise 5-150 KU/l, ist; die Konzentration der Sarcosinoxidase 0,5-150 KU/l, vorzugsweise 2-100 KU/l, ist; und die Konzentration der Peroxidase 1-100 KU/l, vorzugsweise 1-50 KU/l, ist.

9.  *In-vitro*-Verwendung des Kits nach einem der vorhergehenden Ansprüche, wobei der erste Reagenssatz und/oder der zweite Reagenssatz ferner eine Pufferlösung umfasst, die aus der Gruppe ausgewählt ist, die aus MOPS-Puffer, Good-Puffer, TRIS-Puffer, Phosphatpuffer und Glycinpuffer, und einer beliebigen Kombination davon besteht.

10. *In-vitro-Verwendung* des Kits nach einem der vorhergehenden Ansprüche, wobei der erste Reagenssatz die folgenden Mittel umfasst: einen Stabilisator, einen Konservierungsstoff, ein Tensid und/oder eine Ascorbinsäureoxidase.

11. *In-vitro-Verwendung* des Kits nach einem der vorhergehenden Ansprüche, wobei der zweite Reagenssatz ferner einen Konservierungsstoff und/oder ein Tensid umfasst.

12. *In-vitro-Verwendung* des Kits nach einem der vorhergehenden Ansprüche, wobei die biologische Probe zuvor von einem Patienten erhalten wurde, dem Calcium-Dobesilat und/oder Etamsylat verabreicht wurde oder verabreicht wird.

13. *In-vitro-Verwendung* des Kits nach einem der vorhergehenden Ansprüche, wobei die Kreatininmenge in der biologischen Probe weniger als 10 %, vorzugsweise weniger als 8 %, im Vergleich zu der Kreatininmenge, die an einer Kontrollprobe gemessen wurde, stärker bevorzugt weniger als 6 % und am stärksten bevorzugt weniger als 4 %, ist, wobei die Kontrollprobe dieselbe wie die biologische Probe ist, jedoch kein Calcium-Dobesilat und Etamsylat enthält.

14. Verfahren zum *In-vitro*-Quantifizieren der Kreatininmenge in einer biologischen Probe, die Dobesilat und/oder Etamsylat enthält,
wobei das Verfahren Folgendes umfasst:

(a) einen ersten Schritt zum:

(a1) einerseits, Mischen eines ersten Reagenssatzes mit der biologischen Probe, und Messen der Absorption $A1_U$ des Gemisches, und
(a2) andererseits, Mischen eines ersten Reagenssatzes mit einem Kalibrierungsmittel, und Messen der Absorption $A1_C$ des Gemisches,

(b) einen zweiten Schritt zum:

(b1) einerseits, Mischen eines zweiten Reagenssatzes mit dem in Schritt (a1) erhaltenen Gemisch und anschließendes Messen der Absorption $A2_U$ des Gemisches,
(b2) andererseits, Mischen eines zweiten Reagenssatzes mit dem in Schritt (a2) erhaltenen Gemisch und anschließendes Messen der Absorption $A2_C$ des Gemisches,

(c) einen Schritt zum Berechnen der Kreatininmenge gemäß der Formel:

$$\text{Kreatininkonzentration in der biologischen Probe} = (A2_U - A1_U) / (A2_C - A1_C) * C_C$$

wobei Cc die Konzentration des Kalibrierungsmittels darstellt, wobei der erste Reagenssatz ein Chromogen enthält, das aus N-(2-hydroxy-3-sulfopropyl)-3,5-dimethoxyanilin oder seinen Salzen, N-Ethyl-N-(2-hydroxy-3-sulfopropyl)-3,5-dimethoxyanilin oder seinen Salzen, und einer Kombination davon ausgewählt ist, und der zweite Reagenssatz 4-Aminoantipyrin enthält.

**Revendications**

1. Utilisation *in vitro* d'une trousse pour quantifier la quantité de créatinine dans un échantillon biologique qui contient du dobésilate de calcium et/ou de l'étamsylate, ladite trousse comprenant un premier ensemble de réactifs et un second ensemble de réactifs,
ledit premier ensemble de réactifs contenant le chromogène choisi parmi la N-(2-hydroxy-3-sulfopropyle)-3,5-diméthoxyaniline ou ses sels, la N-éthyle-N-(2-hydroxy-3-sulfopropyle)-3,5-diméthoxy-aniline ou ses sels, et des combinaisons de celles-ci, et ledit second ensemble de réactifs contenant de la 4-aminoantipyrine.

**2.** Utilisation *in vitro* de la trousse selon la revendication 1, dans laquelle ledit chromogène est un sel de sodium N-éthyle-N-(2-hydroxy-3-sulfopropyle)-3,5-diméthoxyaniline ou un sel de sodium N-(2-Hydroxy-3-sulfopropyle)-3,5-diméthoxyaniline.

**3.** Utilisation *in vitro* de la trousse selon l'une quelconque des revendications précédentes, dans laquelle ledit premier ensemble de réactifs contient en outre de la créatinase, de la sarcosine oxydase et de la catalase, et ledit second ensemble de réactifs contient en outre de la créatininase et de la peroxydase.

**4.** Utilisation *in vitro* de la trousse selon la revendication 1 ou 2, dans laquelle la concentration de 4-aminoantipyrine est de 0,25 à 5 g/L, de préférence de 0,5 à 2,5 g/L.

**5.** Utilisation *in vitro* de la trousse selon l'une quelconque des revendications précédentes, dans laquelle la concentration de créatininase est de 105 à 800 kU/L, de préférence de 120 à 500 kU/L.

**6.** Utilisation *in vitro* de la trousse selon l'une quelconque des revendications précédentes, dans laquelle la concentration de catalase est de 20 à 1 000 kU/L, de préférence de 50 à 600 kU/L.

**7.** Utilisation *in vitro* de la trousse selon l'une quelconque des revendications précédentes, dans laquelle la concentration en chromogène est de 0,1 à 60 mmol/L, de préférence de 0,3 à 50 mmol/L.

**8.** Utilisation *in vitro* de la trousse selon l'une quelconque des revendications précédentes, dans laquelle la concentration de créatinase est de 1 à 300 kU/L, de préférence de 5 à 150 kU/L ; la concentration de sarcosine oxydase est de 0,5 à 150 kU/L, de préférence de 2 à 100 kU/L ; et la concentration de la peroxydase est de 1 à 100 kU/L, de préférence de 1 à 50 kU/L.

**9.** Utilisation *in vitro* de la trousse selon l'une quelconque des revendications précédentes, dans laquelle ledit premier ensemble de réactifs et/ou ledit second ensemble de réactifs comprend en outre une solution tampon choisie dans le groupe constitué par le tampon MOPS, le tampon de Good, le tampon TRIS, le tampon phosphate et le tampon glycine, et toute combinaison de ceux-ci.

**10.** Utilisation *in vitro* de la trousse selon l'une quelconque des revendications précédentes, dans laquelle ledit premier ensemble de réactifs comprend en outre un ou plusieurs des agents suivants : un stabilisateur, un conservateur, un surfactant et un acide ascorbique oxydase.

**11.** Utilisation *in vitro* de la trousse selon l'une quelconque des revendications précédentes, dans laquelle ledit second ensemble de réactifs comprend en outre un agent conservateur et/ou un surfactant.

**12.** Utilisation *in vitro* de la trousse selon l'une quelconque des revendications précédentes, dans laquelle ledit échantillon biologique a été préalablement prélevé chez un patient qui a reçu une administration ou est en cours d'administration de dobésilate de calcium et/ou d'étamsylate.

**13.** Utilisation *in vitro* de la trousse selon l'une quelconque des revendications précédentes, dans laquelle la quantité de créatinine dans ledit échantillon biologique est inférieure à 10 %, de préférence inférieure à 8 %, comparativement à la quantité de créatinine mesurée par rapport à un échantillon témoin et plus préférablement inférieure à 6 % et idéalement inférieure à 4 %, l'échantillon témoin étant le même que ledit échantillon biologique mais ne contenant pas de dobésilate de calcium ni d'étamsylate.

**14.** Procédé de quantification *in vitro* de la quantité de créatinine dans un échantillon biologique contenant du dobésilate et/ou de l'étamsylate, ledit
procédé comprenant :

(a) une première étape :

(a1) d'une part, mélanger un premier ensemble de réactifs avec ledit échantillon biologique, et mesurer l'absorbance $A1_U$ du mélange, et
(a2) d'autre part, mélanger un premier ensemble de réactifs avec un agent d'étalonnage, et mesurer l'absorbance $A1_C$ du mélange,

(b) une seconde étape :

(b1) d'une part, mélanger un second ensemble de réactifs avec le mélange obtenu à l'étape (a1) puis mesurer l'absorbance $A2_U$ dudit mélange,
(b2) d'autre part, mélanger un second ensemble de réactifs avec le mélange obtenu à l'étape (a2) puis mesurer l'absorbance $A2_C$ dudit mélange,

(c) une étape de calcul de la quantité de créatinine selon la formule :

$$\text{concentration de créatinine dans ledit échantillon biologique} = (A2_U-A1_U)/(A2_C-A1_C) * C_C$$

dans laquelle $C_C$ représente la concentration dudit agent d'étalonnage, le premier ensemble de réactifs contenant un chromogène choisi parmi la N-(2-hydroxy-3-sulfopropyle)-3,5-diméthoxyaniline ou ses sels, la N-éthyle-N-(2-hydroxy-3-sulfopropyle)-3,5-diméthoxyaniline ou ses sels, et des combinaisons de celles-ci, et le second ensemble de réactifs contenant de la 4-aminoantipyrine.